(19) Europäisches Patentamt
European Patent Office
Office européen des brevets

(11) **EP 4 328 236 A1**

(12) **EUROPEAN PATENT APPLICATION**
published in accordance with Art. 153(4) EPC

(43) Date of publication:
**28.02.2024 Bulletin 2024/09**

(21) Application number: **22791804.2**

(22) Date of filing: **22.04.2022**

(51) International Patent Classification (IPC):
*C07K 14/165* (2006.01)     *A61K 39/215* (2006.01)
*C12N 15/50* (2006.01)

(52) Cooperative Patent Classification (CPC):
**A61K 39/215; C07K 14/165**

(86) International application number:
**PCT/JP2022/018507**

(87) International publication number:
**WO 2022/225033 (27.10.2022 Gazette 2022/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.04.2021 US 202163178346 P**

(71) Applicant: **OSAKA UNIVERSITY
Suita-shi
Osaka 565-0871 (JP)**

(72) Inventors:
• **KUROSAKI, Tomohiro
Suita-shi, Osaka 565-0871 (JP)**
• **SHINNAKASU, Ryo
Suita-shi, Osaka 565-0871 (JP)**
• **SAKAKIBARA, Shuhei
Suita-shi, Osaka 565-0871 (JP)**

(74) Representative: **Hoffmann Eitle
Patent- und Rechtsanwälte PartmbB
Arabellastraße 30
81925 München (DE)**

Remarks:
The complete document including Reference
Table(s) and the Sequence Listing(s) can be
downloaded from the EPO website

(54) **GLYCOSYLATED RBD AND USE THEREOF**

(57)     As a technique for efficiently producing anti-coronavirus antibodies with cross-reactivity, provided is a polypeptide comprising an amino acid sequence having at least 60% or more identity to the amino acid sequence of SEQ ID NO: 2, wherein the amino acid sequence is modified so as to allow sugar chain attachment to positions corresponding to positions 85, 164, and 172 of the amino acid sequence of SEQ ID NO: 2.

Fig. 2-1

A     Side view     Top view

RBD { Head Core

**(Cont. next page)**

EP 4 328 236 A1

Fig. 2-1

## B

| AA number | Added glycans | | | | |
|---|---|---|---|---|---|
| | 415 | 448 | 477 | 494 | 502 |
| WT | TGK | NYN | STP | SYG | GVG |
| GM9 | NGT | | | NYT | NVT |
| GM14 | NGT | NYT | NTT | NYT | NVT |

**Description**

Technical Field

Disclosed are techniques relating to sugar chain modification of the receptor-binding domain (RED) of SARS-CoV-2 spike protein.

Background Art

The COVID-19 pandemic, caused by the β-coronavirus severe acute respiratory syndrome coronavirus 2 (SARS-CoV-2), is a global health crisis. Coronavirus entry into host cells is mediated by the virus S protein, which forms trimeric spikes on the viral surface. The entry receptor for SARS-CoV-2 and SARS-CoV (herein called SARS-CoV-1) is the human cell-surface angiotensin converting enzyme 2 (ACE2), and the receptor-binding domain (RED) of the spike from both of these viruses binds ACE2 with high affinity. Hence, it is believed that the RBD can become a primary target for neutralizing antibodies.
However, given that prior coronavirus epidemics (e.g., SARS-CoV-1 and MERS-CoV) have occurred due to zoonotic coronaviruses crossing the species barrier (Non-patent Literature (NPL) 1), the potential for the emergence of similar viruses in the future poses a significant threat to global public health, even in the face of effective vaccines for current viruses. For instance, one pre-emergent bat coronavirus, WIV1-CoV, is highly homologous to SARS-CoV-1 and SARS-CoV-2 and has been shown to be able to infect human ACE2-expressing cells (NPL 2). However, sera from SARS-CoV-2 infected individuals have shown very limited cross-neutralization of WIV1-CoV, except for rare individuals with low-levels of neutralizing antibodies (NPL 3).

Citation List

Non-patent Literature

NPL 1: Wacharapluesadee, S., Tan, C.W., Maneeom, P., Duengkae, P., Zhu, F., Joyjinda, Y., Kaewpom, T., Chia, W.N., Ampoot, W., Lim, B.L., et al. (2021). Evidence for SARS-CoV-2 related coronaviruses circulating in bats and pangolins in Southeast Asia. Nat Commun 12, 972.
NPL 2: Menachery, V.D., B.L. Yount Jr., K. Debbink, S. Agnihothram, L.E. Gralinski, J.A. Plante, R.L. Graham, T. Scobey, X.Y. Ge, E.F. Donaldson, et al. 2015. A SARS-like cluster of circulating bat coronaviruses shows potential for human emergence. Nat. Med. 21: 1508-1513.
NPL 3: Garcia-Beltran, W.F., Lam, E.C., Astudillo, M.G., Yang, D., Miller, T.E., Feldman, J., Hauser, B.M., Caradonna, T.M., Clayton, K.L., Nitido, A.D., et al. (2021). COVID-19-neutralizing antibodies predict disease severity and survival. Cell 184, 476-488 e411.

Summary of Invention

Technical Problem

An object is to provide a technique for obtaining an anti-coronavirus antibody with cross-reactivity.

Solution to Problem

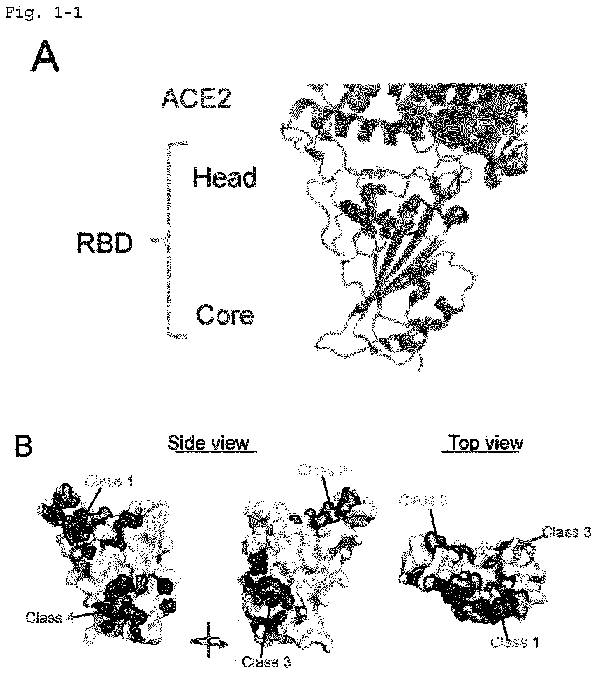

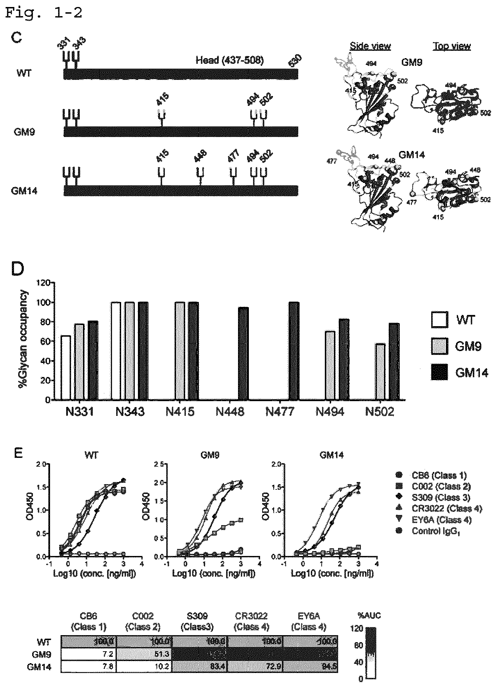

The SARS-CoV-2 RBD is composed of two subdomains (Fig. 1A). The core-RBD subdomain consists of a central β sheet flanked by α-helices and presents a stable folded scaffold for the receptor-binding motif (RBM, residues 437-508) (Shang et al., 2020, Structural basis of receptor recognition by SARS-CoV-2. Nature 581, 221-224), which encodes ACE2 binding and receptor specificity (herein, the RBM-encoded region is called the head-RBD subdomain) (Shang et al., 2020, the same as above). Although initial analysis of convalescent sera from SARS-Cov-2-infected individuals demonstrated that many neutralizing antibodies (Class 1 and 2) recognize the head-RBD subdomain (Fig. 1B), cross-neutralization of known antibodies is very limited, as stated above. The present inventors thus conducted extensive research and found, in terms of broadly protective responses, that the core-RBD subdomain could be a more promising target.
One of the potential obstacles to targeting the core-RBD subdomain is that the epitopes in this subdomain are immuno-subdominant. Possible approaches to circumvent this problem include at least the following: i) altering the immunogen surface through targeted point mutations and deletions (Jardine et al., 2013, Rational HIV immunogen design to target specific germline B cell receptors. Science 340, 711-716; Impagliazzo et al., 2015, A stable trimeric influenza hemagglutinin stem as a broadly protective immunogen. Science 349, 1301-1306; Valkenburg et al., 2016, Stalking influenza by vaccination with pre-fusion headless HA mini-stem. Sci Rep 6, 22666); and ii) introducing N-linked glycans believed to shield the neighboring epitopes by means of the NxS/T sequons (Duan et al., 2018, Glycan Masking Focuses Immune Responses to the HIV-1 CD4-Binding Site and Enhances Elicitation of VRC01-Class Precursor Antibodies. Immunity 49, 301-311 e305; Eggink et al., 2014, Guiding the immune response against influenza virus hemagglutinin toward the conserved stalk domain by hyperglycosylation of the globular head domain. J Virol 88, 699-704). To facilitate the immune responses to the core-RBD subdomain, the present inventors introduced glycans into specific sites in the SARS-CoV-2 head-RBD subdomain, demonstrating that the glycan engineering facilitated the elicitation of potent cross-neutralizing antibodies towards SARS-CoV-1 and WIV1-CoV (clade 1 sarbecoviruses). The inventors conducted further study and research based on these findings to thus provide the subject matter represented by the following.

Item 1. A polypeptide comprising an amino acid sequence having at least 60% or more identity to the amino acid sequence of SEQ ID NO: 2, wherein the amino acid sequence is modified so as to allow sugar chain attachment to positions corresponding to positions 85, 164, and 172 of the amino acid sequence of SEQ ID NO: 2.

Item 2. The polypeptide according to Item 1, wherein the modified amino acid sequence is further modified so as to allow sugar chain attachment to a position corresponding to position 118 and/or a position corresponding to position 147 of the amino acid sequence of SEQ ID NO: 2.

Item 3. The polypeptide according to Item 1 or 2, wherein the sugar chain is an N-type sugar chain.

Item 4. The polypeptide according to any one of Items 1 to 3, wherein the sugar chain is attached to each of positions corresponding to positions 85, 164, and 172 of the amino acid sequence of SEQ ID NO: 2.

Item 5. The polypeptide according to Item 4, wherein a sugar chain is further attached to a position corresponding to position 118 and/or a position corresponding to position 147.

Item 6. The polypeptide according to Item 4 or 5, wherein the sugar chain is an N-type sugar chain.

Item 7. A polynucleotide comprising a base sequence encoding the polypeptide of Item 1 or 2.

Item 8. A composition comprising the polypeptide of any one of Items 1 to 6 or the polynucleotide of Item 7.

Item 9. The composition according to Item 8, which is a vaccine.

Item 10. A method for producing an anti-coronavirus antibody, comprising immunizing an animal with the polypeptide of any one of Items 1 to 6.

Item 11. An antibody obtained by the method of Item 10.

Advantageous Effects of Invention

Provided are techniques for efficiently producing anti-coronavirus antibodies with cross-reactivity.

Brief Description of Drawings

Fig. 1 shows design of SARS-CoV-2 RBD glycan-masked mutants. (A) Structure of RBD of CoV-2 S (PDB: 6YZ5) that engages the ACE-2 ectodomain (PDB: 6M0J). (B) Epitopes of representative anti-CoV-2 mAbs. (C) (left) Glycosylation sites in RBD WT, GM9, and GM14. (C) (right) Ribbon models of RBD GM9 and GM14. The spheres are the introduced N-type glycan sites. (D) Glycan occupancy at N-linked glycosylation sites of RBD WT, GM9, and GM14 determined by LC-MS. (E) ELISA binding of previously reported mAbs against SARS-CoV-2 RBD (CB6, C002, S309, CR3022, and EY6A) to WT, GM9 and GM14. Anti-C. albicans hIgG1 mAb 23B12 was used for a negative control. The heatmap shows the percentage of binding (the value of the area under the curve [AUC] in ELISA for RBD WT is set at 100% for each mAb). Representative results from three independent experiments are shown.

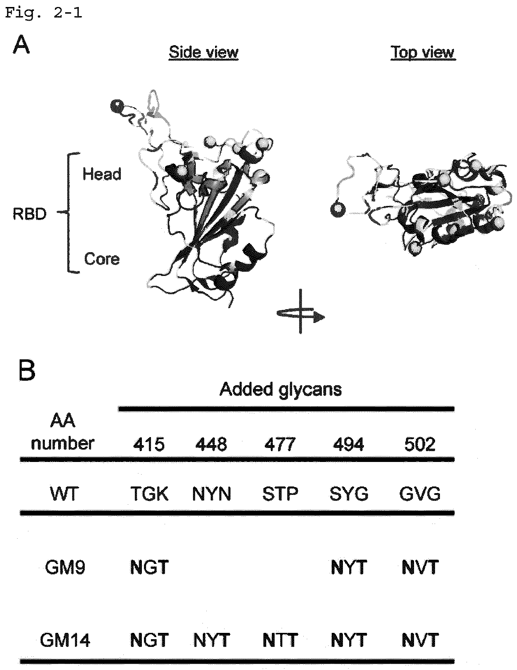

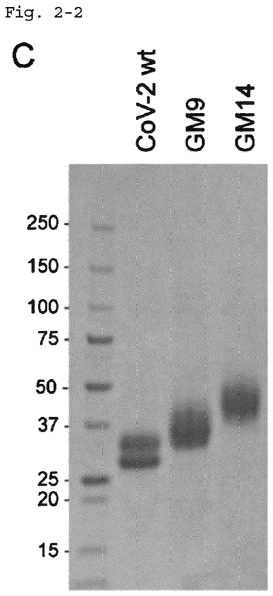

Fig. 2 shows design and expression of CoV-2 RBD glycan-masked mutants. (A) Structure of RBD-ACE2 complex. (B) Parental amino acid sequences and introduced NXT sequons. (C) SDS-PAGE of RED WT, GM9, and GM14.

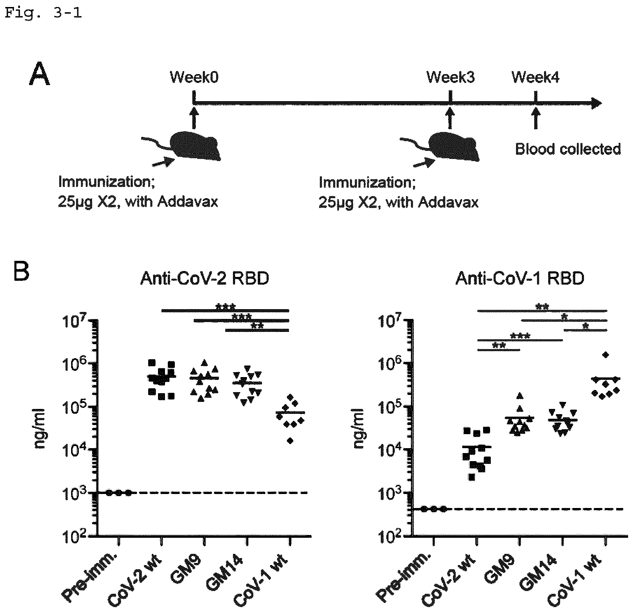

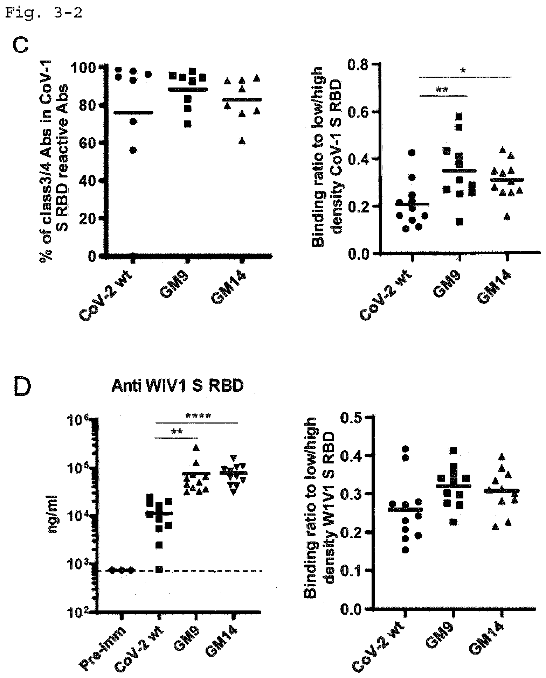

Fig. 3 shows that glycan mutants of CoV-2 spike RBD immunogen effectively elicited CoV-1 S RBD specific antibodies. (A) Schematic overview of the experimental design. (B) CoV-2 RBD (left) or CoV-1 RBD (right) specific serum IgG1 antibody levels in serum from pre-immune mice, or CoV-2 RBD, GM9, GM14, or CoV-1 RBD WT immunized mice were measured by ELISA. Samples were pooled from three independent experiments (3-4 mice each group). (C) Class 3/4 type serum antibodies from CoV-2 RBD, GM9, GM14, or CoV-1 RBD WT immunized mice were measured by Epitope-Blocking ELISA with a mixture of CR3022/EY6A/S309 human IgG1 mAbs (left). Affinity measurements were determined by ELISA, expressed as the binding ratio to low density/high density of plate bound CoV-1 RBD protein (right). (D) WIV1 RBD-specific serum IgG1 antibody levels (left) and affinity (right) were measured as in (B) and (C, right), respectively. Horizontal lines indicate mean values; each symbol indicates one mouse. *, p < 0.05; **, p < 0.01; ***, p < 0.001; unpaired Student's t-test.

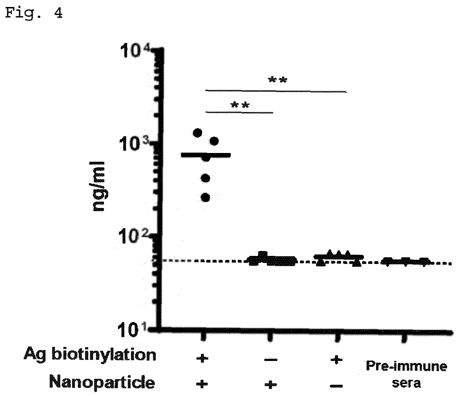

Fig. 4 shows that immunization with a nanoparticle antigen leads to the effective induction of CoV-2 S RBD specific antibodies. ELISA plots for CoV-2 RBD wt probe recognition of serum from respective biotin (+) RBD/St-avi nanoparticles, biotin (-) RBD/St-avi nanoparticles or only biotin (+) RBD immunized mice 3 weeks post primary immunization. Horizontal lines indicate mean values; each symbol indicates one mouse. The CR3022 mouse IgG1 monoclonal antibody (InvivoGen) was used as a standard. *, p < 0.05; **, p < 0.01; ***, p < 0.001; unpaired Student's t-test.

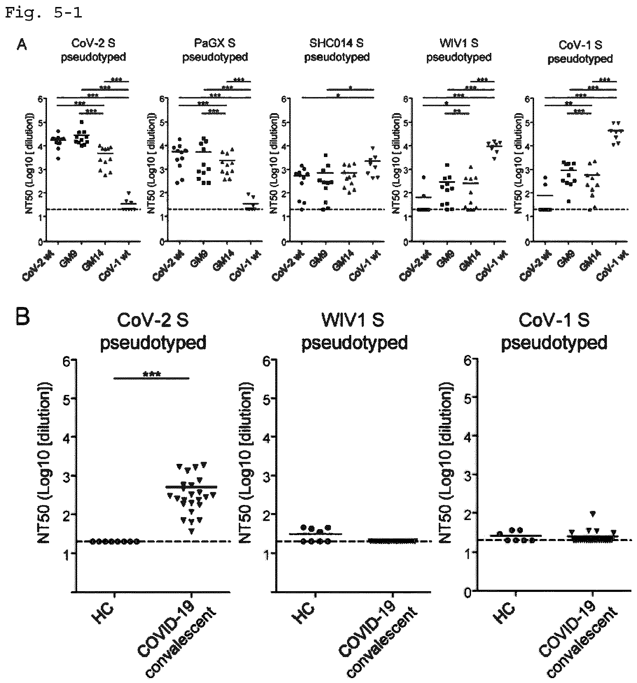

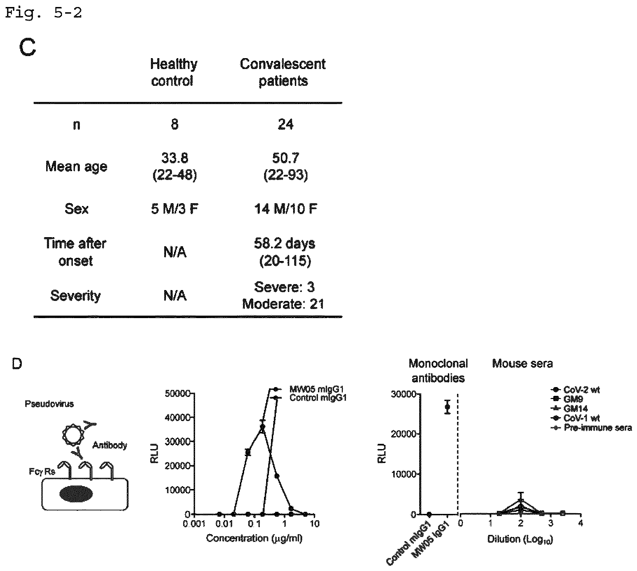

Fig. 5 shows that glycan engineering of the SARS-CoV-2 head-RBD elicited cross-neutralizing activities against SARS-CoV-1 and WIV1-CoV. (A) BALB/c mice were prime-boost-immunized with CoV-2 RED WT, GM9, GM14 or CoV-1 RED as shown in Fig. 3. Sera were collected 7 days after boost and pre-incubated with CoV-2 S, CoV-1 S, or WIV-1 CoV S-pseudotyped VSV∆G-luc for 1 h. The mixture was incubated with VeroE6 TMPRESS2 cells overnight. The NT50s of each group of mice are shown in the graphs. NT50s of the sample below the detection limit (1:20) was set as 20. *P< 0.05, Wilcoxon test. RED WT (n = 11); GM9 (n = 11); GM14 (n = 11); CoV-1 RBD (n = 8). (B) Neutralization activities of plasma samples from pre-pandemic healthy donors (HC, n = 8) or convalescent COVID-19 patients (n = 24) against CoV-2, CoV-1 and WIV-1 CoV S-pseudotyped VSV∆G-luc. NT50s of the sample below the detection limit (1:20) was set as 20. (C) Donor information. (D) ADE of infection by DENV1 single-round infectious particles (SRIP) by anti-DENV1 E mAb 3H12. DENV1 SRIP were pre-incubated with different concentrations of 3H12 or control mouse IgG1 and then incubated with RAW264.7 cells. The luciferase activity was measured 72 h post infection. (E) Sera of immunized mice were serially diluted and mixed with CoV-2 S pseudotyped VSV∆G-luc for 1 h and incubated with RAW264.7 cells. The luciferase activity was measured 24 h post infection. Representative results from two to three independent experiments are shown. Data are mean ± SEM. Dotted lines in the graphs (NT50 = 20) represent the lower limit of detection (A and B). *, p < 0.05; **, p < 0.01; ***, p < 0.001; unpaired Student's t-test.

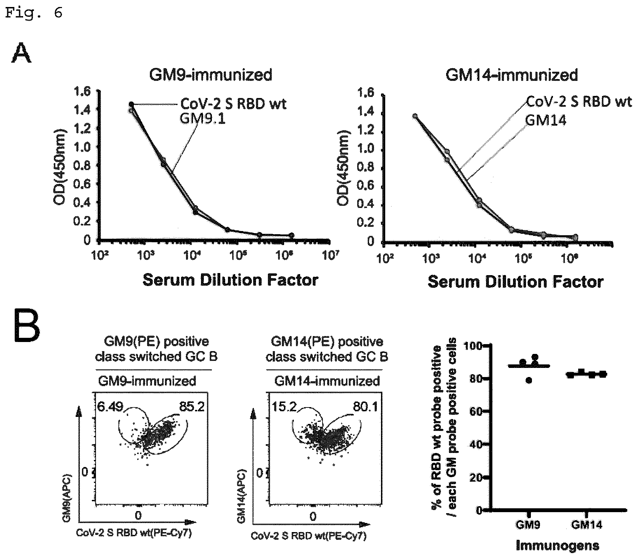

Fig. 6 shows cross-reactivity of sera and GC B cells in mice immunized with GM9 or GM14. (A) ELISA plots for CoV-2 RBD wt probe recognition of serum of mice after prime/boost with GM9 or GM14. Sera were collected at 3 weeks post primary immunization. (B) Representative FACS plots of GC B from dLNs after immunization. Cells were gated for antigen-binding IgG+ GC B cells (CD138-B220+IgG+GL7+Fas+). The graph (right) shows the percentage of positive cells for RBD wt binding among GM9 or GM14-binding cells (n=4 each).

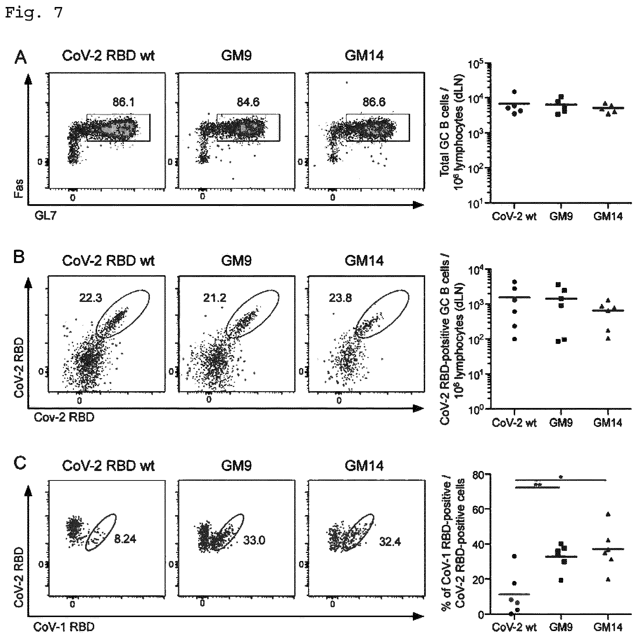

Fig. 7 shows that immunization with CoV-2 GM antigens leads to the activation of CoV-1 and CoV-2 cross-reactive GC B cells. (A) Representative flow cytometry plots analyzing GC B cells (CD138-B220+IgG+GL7+Fas+) from lymph nodes of mice 3 weeks post primary immunization with each antigen. The plots are representative of findings from 2 independent experiments (left). Quantification of absolute numbers of CD138-B220+IgG+GL7+Fas+ GC B cells (right) from multiple mice in one experiment. Results are representative of 2 independent experiments. Horizontal lines indicate mean values; each symbol indicates one mouse. (B) Representative analytical results by flow cytometry of CoV-2 RBD-binding MLN GC B cells (CD138-B220+IgG+GL7+Fas+CoV-2 RBD(APC)+ CoV-2 RBD. Plots are representative of findings from 2 independent experiments (left). Absolute numbers of GC B cells (right) from multiple mice in one experiment are shown. Results are representative of 2 independent experiments. (C) Cross-reactivity of CoV-2 RED-binding MLN GC B cells (CD138-B220+IgG+GL7+Fas+CoV-2 RBD(APC)+ CoV-2 RED(PE-Cy7+) with CoV-1 RBD assessed by flow cytometry. Plots are representative of findings from 2 independent experiments (left). The frequency of CoV-1 and CoV-2 RBD cross-reactive GC B cells (right) from multiple mice in one experiment. Results are representative of 2 independent experiments. Horizontal lines indicate mean values; each symbol indicates one mouse. *, P < 0.05; **, P < 0.01; ***, P < 0.001; unpaired Student's t-test.

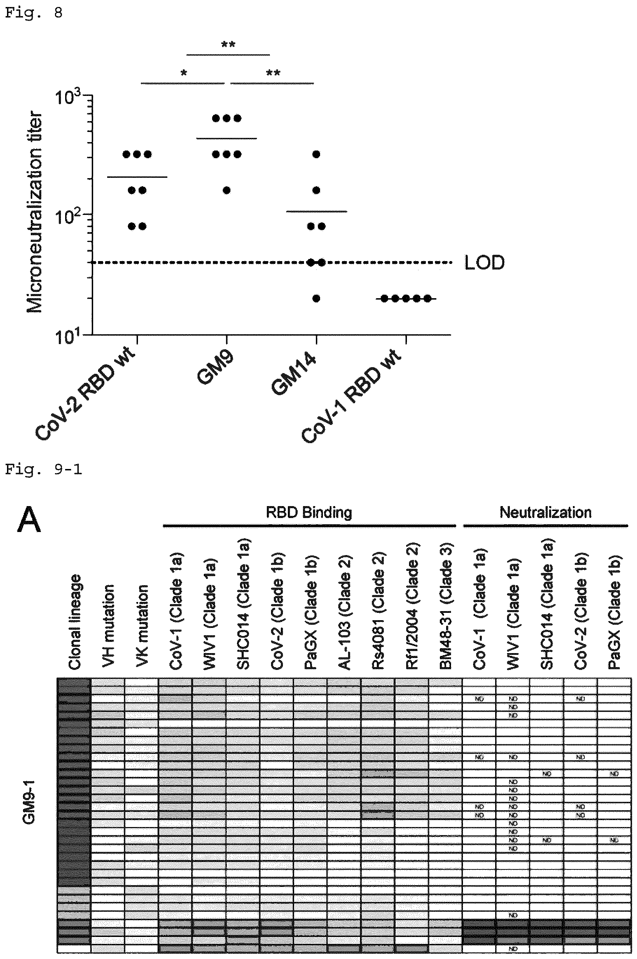

Fig. 8 shows authentic virus neutralizing assay. A mixture of 100 TCID50 virus and serially diluted, heat-inactivated plasma samples were incubated for 1 h before being placed on VeroE6-TMPRSS2 cells seeded in 96-well plates. After culturing for 4 days, cells were fixed with formalin and stained with crystal violet solution. Cut-off dilution index with >50% cytopathic effect was presented as microneutralization titer. Microneutralization titer of the sample below the detection limit (1:40 dilution) was set as 20. *, P < 0.05; **, P < 0.01; unpaired Student's t-test.

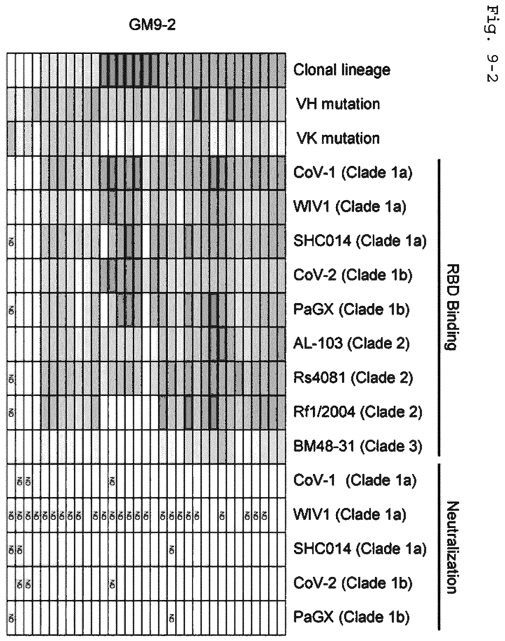

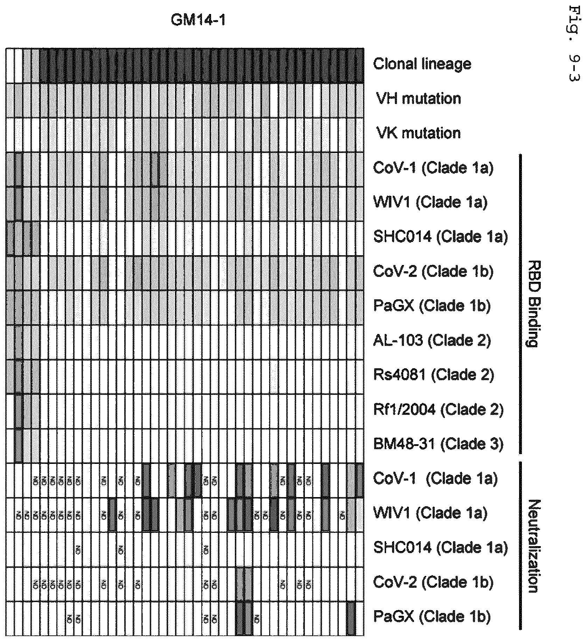

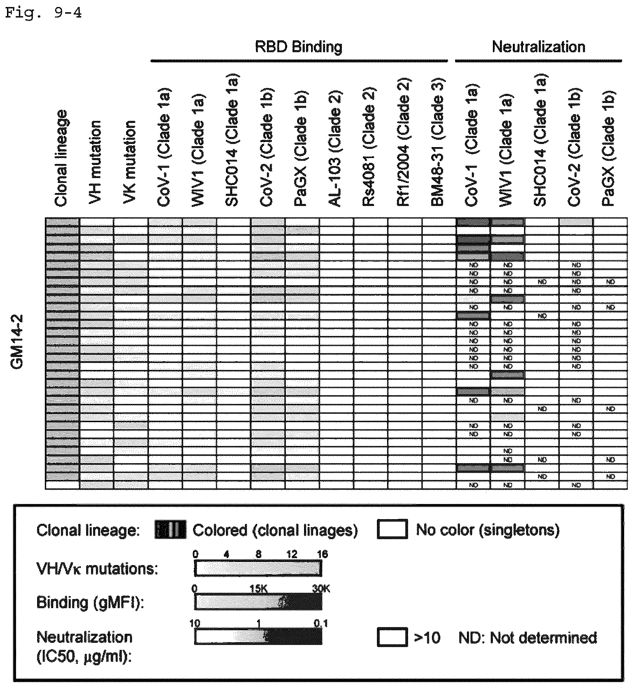

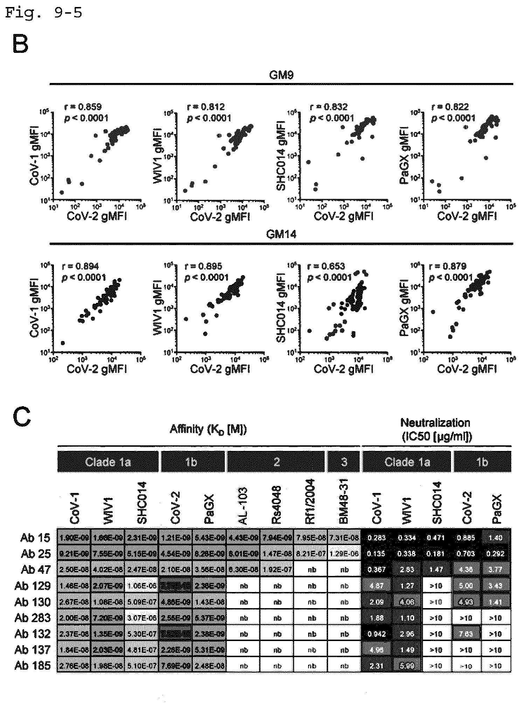

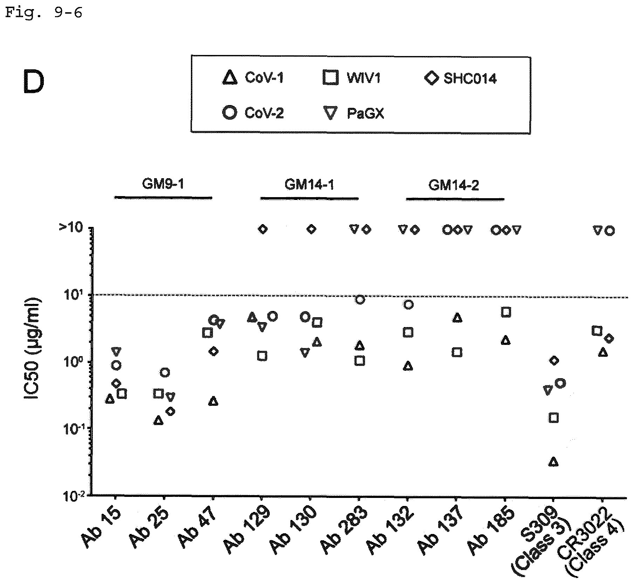

Fig. 9 shows that GC-derived mAbs exhibited breadth of RBD binding and neutralization against clade 1 SARS-related CoVs.

(A) Clonality, VH and VK mutations and antibody class, binding to S REDs of CoV-1, WIV1-CoV, CoV-2, BatCoV Anlong-103 (AL-103) and BtCoV BM48-31, neutralizing activity against CoV-1, WIV1-CoV and CoV-2 pseudoviruses of mAbs derived from GC B cells of mice immunized with GM9 (GM9-1 and GM9-2) or GM14 (GM14-1 and GM14-2) .

(B) Correlation between mAb binding to CoV-2 RBD and CoV-1 or WIV1-CoV RBD. Binding of mAbs with CoV-1, WIV1 and CoV-2 RBDs were measured by bead-based flow cytometric assays. The binding signals to respective REDs (gMFI) are plotted in the graph. Spearman's rank correlation coefficients and p values are shown.

(C) Binding affinity of mAbs with RBD proteins. The equilibrium dissociation constants (M) and $IC_{50}$s ($\mu$g/ml)

of individual clones are shown. (D) Neutralizing activity (IC$_{50}$ [μg/ml] ) of representative cross-reactive mAbs and mouse IgG2c recombinant antibodies carrying the variable regions of previously isolated S304 (class 3) and CR3022 (class 4) human anti-RED antibodies.

Fig. 10 shows correlation between gMFI from bead-based flow-cytometry assay and RMax from biolayer interferometry (Octet) assay. gMFI and KD(M), RMax, kon (1/Ms) and koff (1/s) of bivalent mAb-CoV-2 RBD interactions are shown. Spearman's rank test.

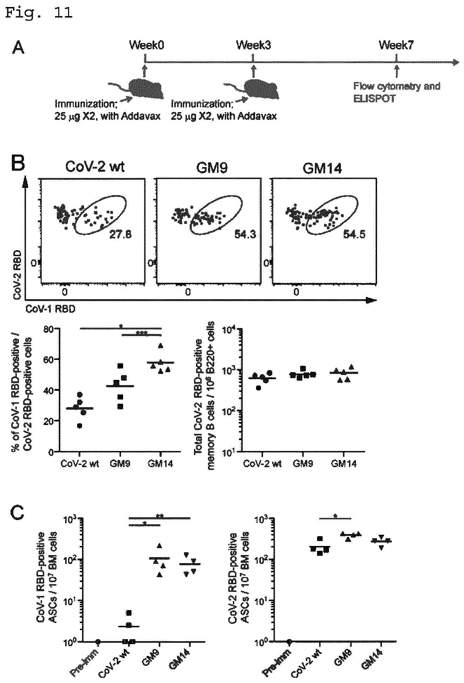

Fig. 11 shows generation of cross-reactive memory B cells and long-lived plasma cells in mice immunized with GM9 or GM14. (A) Schematic overview of the experimental design. (B) Cross-reactivity of CoV-2 RBD-specific memory B cells (CD138-B220+IgG1+CD38+GL7-CoV-2 RED(APC)+CoV-2 RBD(PE-Cy7)+) with CoV-1 RBD from spleen of mice one month post boost immunization with each antigen. Frequency of CoV-1 and CoV-2 RBD cross-reactive memory B cells (upper) and quantification of absolute numbers of total CoV-2-specific memory B cells (lower) from multiple mice in one experiment. Results are representative of 2 independent experiments. (C) ELISPOT analysis of CoV-1- (left) or CoV-2-(right) specific IgG1 ASC responses of pre-immune mice, or one month post boost immunizations with each antigen. Results are representative of 2 independent experiments. Horizontal lines indicate mean values; each symbol indicates one mouse. *, P < 0.05; **, P < 0.01; ***, P < 0.001; unpaired Student's t-test.

Fig. 12 shows characteristics regarding mAb generated from GC B cells of mice immunized with GM9 or GM14.

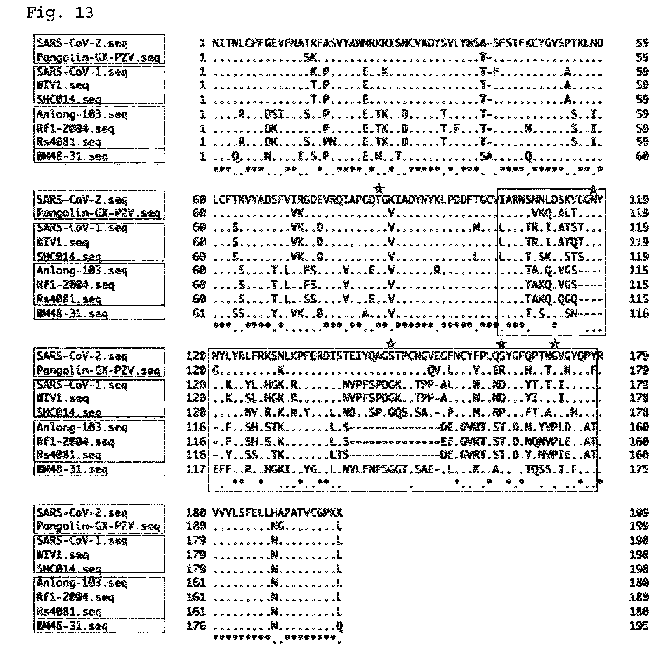

Fig. 13 shows comparisons among the RBD sequences of representative sarbecoviruses of the genus β-coronavirus. The regions enclosed by squares are receptor binding motifs (RED head regions). The star symbols represent sites into which the sugar chain modification was inserted. The RBD amino acid sequences used for the alignment in Fig. 13 are SEQ ID NOs: 2, 107, 108, 109, 110, 111, 112, 113, and 114 in this order from the top.

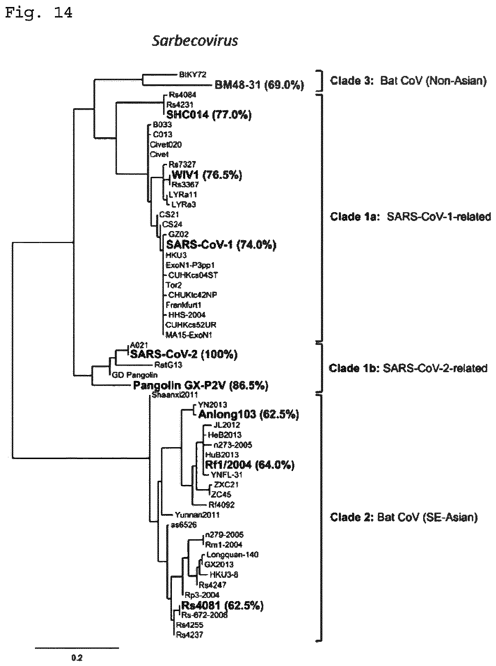

Fig. 14 shows a phylogenetic tree of RBD sequences of representative sarbecoviruses of the genus β-coronavirus. The numerical values in parentheses indicate homology to SARS-Cov-2 RBD.

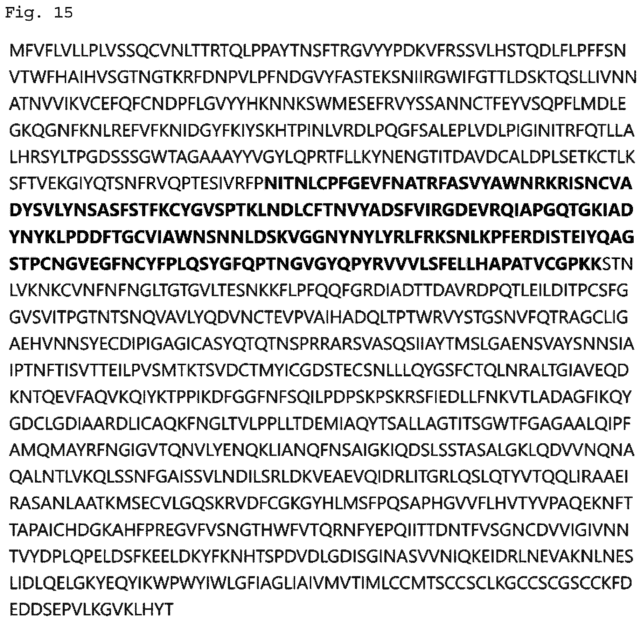

Fig. 15 shows the amino acid sequence (SEQ ID NO: 1) of the SARS-CoV-2 spike protein. The region shown in bold is the receptor binding domain (SEQ ID NO: 2).

Description of Embodiments

1. Definition

In the present specification, the terms "comprising," "containing," and "having" include the concepts of containing, including, consisting essentially of, and consisting of.

In the present specification, the "identity" of amino acid sequences refers to the degree of identicalness of two or more amino acid sequences that can be compared with each other. Thus, the higher the identicalness of two amino acid sequences, the higher the identity or similarity of these sequences. The level of amino acid sequence identity is determined, for example, using FASTA, which is a tool for sequence analysis, with default parameters. The level of amino acid sequence identity can otherwise be determined using the algorithm BLAST by Karlin and Altschul. A program called "BLASTX," which is based on such an algorithm of BLAST, has been developed. Specific procedures of these analysis methods are known, and reference may be made to the website (http://www.ncbi.nlm.nih.gov/) of the National Center of Biotechnology Information (NCBI). The "identity" of base sequences is also defined accordingly.

In the present specification, the term "corresponding positions" in terms of amino acid sequences and nucleotide sequences can be determined by aligning (alignment of) a target sequence and a reference sequence (e.g., the amino acid sequence of SEQ ID NO: 2) so as to achieve maximum homology between the residues present in each sequence. Alignment can be conducted by using known algorithms, and the procedures thereof are known to those skilled in the art. For example, alignment can be performed by using the Clustal W multiple alignment program (Nucleic Acids Res, 1994, 22:4673-4680) with default settings. Alternatively, Clustal W2 or Clustal omega, revised versions of Clustal W, can also be used. Clustal W, Clustal W2, and Clustal omega are available, for example, on the websites of the European Bioinformatics Institute (EBI [www.ebi.ac.uk/index.html]) and the DNA Data Bank of Japan operated by the National Institute of Genetics (DDBJ [www.ddbj.nig.ac.jp/Welcome-j.html]).

In the present specification, "conservative substitution" means a substitution of an amino acid residue with an amino acid residue having a similar side chain. For example, a substitution between amino acid residues having a basic side chain, such as lysine, arginine, and histidine, is considered to be a conservative substitution. Other examples that are considered to be a conservative substitution include a substitution between amino acid residues having an acidic side chain, such as aspartic acid and glutamic acid; a substitution between amino acid residues having an uncharged polar side chain, such as glycine, asparagine, glutamine, serine, threonine, tyrosine, and cysteine; a substitution between amino acid residues having a nonpolar side chain, such as alanine, valine, leucine, isoleucine, proline, phenylalanine, methionine, and tryptophan; a substitution between amino acid residues having a β-branched side chain, such as threonine, valine, and isoleucine; and a substitution between amino acid residues having an aromatic side chain, such as tyrosine, phenylalanine, tryptophan, and histidine.

2. Polypeptide

The polypeptide preferably has a certain degree or more of identity to the amino acid sequence of SEQ ID NO: 2. The certain degree or more means, for example, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more.

SEQ ID NO: 2 is the amino acid sequence of RBD composed of 199 amino acid residues at positions 331 to 529 of the amino acid sequence of SARS-CoV-2 S protein (SEQ ID NO: 1). In one embodiment, the polypeptide may be shorter than 199 residues in length or longer than 199 amino acid residues in length. In one embodiment, the polypeptide is 1 residue, 2 residues, 3 residues, 4 residues, 5 residues, 6 residues, 7 residues, 8 residues, 9 residues, or 10 residues longer than 199 residues in length, or 1 residue, 2 residues, 3 residues, 4 residues, 5 residues, 6 residues, 7 residues, 8 residues, 9 residues, or 10 residues shorter than 199 residues in length. When the polypeptide is longer than 199 residues in length, the longer sequence portion preferably has a certain degree or more of identity to the corresponding amino acid sequence of SEQ ID NO: 1. The meaning of the phrase "certain degree or more" used here is as described above. In one embodiment, the polypeptide may comprise, for example, a Tag sequence at the C- or N-terminus.

In one embodiment, if the amino acid sequence of the polypeptide does not exactly match the amino acid sequence of SEQ ID NO: 1 or 2, the mismatched amino acid residues are preferably based on conservative amino acid substitutions in the corresponding amino acid residues in the amino acid sequence of SEQ ID NO: 1 or 2.

The polypeptide preferably has an amino acid sequence that is modified so as to allow sugar chain attachment to positions corresponding to positions 85, 164 and 172 of the amino acid sequence of SEQ ID NO: 2. The type of sugar chain is not limited and may be either an N-type sugar chain or O-type sugar chain. In one embodiment, the sugar chain is preferably an N-type sugar chain.

Techniques for sugar chain attachment to specific positions of a polypeptide are known. For example, for N-type sugar chain attachment to a position corresponding to position 85 of the amino acid sequence of SEQ ID NO: 2, the amino acid residue corresponding to position 85 of the amino acid sequence of SEQ ID NO: 2 is preferably an aspartic acid residue while the amino acid residue corresponding to position 87 is preferably a serine residue or a threonine residue (preferably a threonine residue). Similarly, for N-type sugar chain attachment to a position corresponding to position 164 of the amino acid sequence of SEQ ID NO: 2, the amino acid residue corresponding to position 164 of the amino acid sequence of SEQ ID NO: 2 is preferably an aspartic acid residue while the amino acid residue corresponding to position 166 is preferably a serine residue or a threonine residue (preferably a threonine residue). Similarly, for N-type sugar chain attachment to a position corresponding to position 172 of the amino acid sequence of SEQ ID NO: 2, the amino acid residue corresponding to position 172 of the amino acid sequence of SEQ ID NO: 2 is preferably an aspartic acid residue while the amino acid residue corresponding to position 174 is preferably a serine residue or a threonine residue.

In one embodiment, the polypeptide preferably has an amino acid sequence that is modified so as to allow sugar chain attachment to a position corresponding to position 118 and/or a position corresponding to position 147, in addition to positions corresponding to positions 85, 164, and 172 of the amino acid sequence in SEQ ID NO: 2. In one embodiment, the polypeptide preferably has an amino acid sequence that is modified so as to allow sugar chain attachment to positions corresponding to positions 118 and 147, in addition to positions corresponding to positions 85, 164, and 172 of the amino acid sequence of SEQ ID NO: 2. In one embodiment, the polypeptide preferably has an amino acid sequence that is modified so as to allow sugar chain attachment to positions corresponding to positions 85, 164, and 172 of the amino acid sequence of SEQ ID NO: 2, wherein the amino acid sequence is not modified so as to allow sugar chain attachment to a position corresponding to position 118 and/or a position corresponding to position 147 of the amino acid sequence of SEQ ID NO: 2. In one embodiment, the polypeptide preferably has an amino acid sequence that is not modified so as to allow sugar chain attachment to at least one position corresponding to at least one position selected from the group consisting of positions 87, 126, 141, 152, 154, and 157 of the amino acid sequence of SEQ ID NO: 2, from the viewpoint of allowing stable expression thereof. The phrase "at least one" used here can mean at least two, at least three, at least four, at least five, or at least six. The type of sugar chain is not limited and may be either an N-type sugar chain or O-type sugar chain. In one embodiment, the sugar chain is preferably an N-type sugar chain.

For N-type sugar chain attachment to a position corresponding to position 118 of the amino acid sequence of SEQ ID NO: 2, the amino acid residue corresponding to position 118 of the amino acid sequence of SEQ ID NO: 2 is preferably an aspartic acid residue, and the amino acid residue corresponding to position 120 is preferably a serine residue or a threonine residue (preferably a threonine residue). Similarly, for N-type sugar chain attachment to a position corresponding to position 147 of the amino acid sequence of SEQ ID NO: 2, the amino acid residue corresponding to position 147 of the amino acid sequence of SEQ ID NO: 2 is preferably an aspartic acid residue, and the amino acid residue corresponding to position 149 is preferably a serine residue or a threonine residue (preferably a threonine residue).

In one embodiment of the polypeptide, in the amino acid sequence of SEQ ID NO: 2, the amino acid residues at positions 85, 164, and 172 are preferably substituted with an aspartic acid residue, and the amino acid residues at positions 87, 166, and 174 are preferably substituted with a serine residue or a threonine residue (preferably a threonine residue). In one embodiment of the polypeptide, in the amino acid sequence of SEQ ID NO: 2, the amino acid residue at positions 85, 118, 147, 164, and 172 are preferably substituted with an aspartic acid residue, and the amino acid residues at positions 87, 120, 149, 166, and 174 are preferably substituted with a serine residue or a threonine residue (preferably, a threonine residue).

In one embodiment, the polypeptide preferably has a certain degree or more of glycan occupancy at one or more positions corresponding to one or more positions selected from the group consisting of positions 85, 118, 147, 164, and 172 of the amino acid sequence of SEQ ID NO: 2. The glycan occupancy can be measured by the method described in the Examples below. The phrase "certain degree or more" means, for example, 30% or more, 40% or more, 50% or more, 60% or more, 70% or more, 80% or more, or 90% or more. In one embodiment, the phrase "one or more" includes two or more, three or more, or four or more.

The polypeptide may be in the form of a pharmaceutically acceptable salt formed with an acid or base. The salt is not limited as long as it is a pharmaceutically acceptable salt, and may be either an acid salt or a basic salt. Examples of acid salts include inorganic acid salts, such as hydrochloride, hydrobromide, sulfate, nitrate, and phosphate; organic acid salts, such as acetate, propionate, tartrate, fumarate, maleate, malate, citrate, methanesulfonate, and para-toluenesulfonate; and amino acid salts, such as aspartate and glutamate. Examples of basic salts include alkali metal salts, such as sodium salts and potassium salts; and alkaline earth metal salts, such as calcium salts and magnesium salts.

The polypeptide may otherwise be in the form of a solvate. The solvent is not limited as long as it is pharmaceutically acceptable, and may be, for example, water, ethanol, glycerol, or acetic acid.

The polypeptide can be easily prepared according to a known genetic engineering method, according to its amino acid sequence. For example, the polypeptide can be prepared using PCR, restriction enzyme cleavage, a DNA ligation technique, an in vitro transcription/translation technique, a recombinant protein production technique, etc. In one embodiment, the polypeptide can be obtained by expressing a polynucleotide encoding the polypeptide in an appropriate host.

The polypeptide may be purified after synthesis. For example, the polypeptide may be obtained by extraction from cells harvested and collected by centrifugation, filtration, etc. from a culture product. Specifically, at the first step of extraction, the cells can be destroyed by means of digestion with an enzyme, destruction caused by osmotic pressure, a sudden increase or decrease in pressure, sonication, various homogenizers, etc. The destroyed cells can then be fractionated by physical means such as low-speed centrifugation, ultra-centrifugation, filtration, molecular sieving, and membrane concentration, chemical means such as a precipitant, a solubilizer, an adsorption/desorption agent, and a dispersant, or physicochemical means such as electrophoresis, column chromatography, a support, dialysis, and salting-out. These techniques may be used in combination. In applying these techniques, physicochemical conditions, such as temperature, pressure, pH, and ion strength, can be suitably set.

The polypeptide is useful for efficiently producing anti-coronavirus antibodies with cross-reactivity. In one embodiment, cross-reactivity means reactivity to SARS-CoV-2 and coronaviruses other than SARS-CoV-2. In one embodiment, the coronaviruses other than SARS-CoV-2 are those belonging to the genus β-coronavirus, and preferably those belonging to the subgenus sarbecovirus of the genus β-coronavirus. In one embodiment, the coronaviruses other than SARS-CoV-2 are SARS-related coronaviruses. In one embodiment, examples of the coronaviruses other than SARS-CoV-2 include SARS-CoV-1, WIV1-CoV, MERS-CoV, and Pangolin-CoV. In one embodiment, the anti-coronavirus antibodies with cross-reactivity are preferably neutralizing antibodies, and preferably broadly neutralizing antibodies. Such antibodies are useful in preventing, treating, or alleviating diseases or conditions caused by coronaviruses. Accordingly, the polypeptide can be used as a vaccine or therapeutic agent for these purposes.

3. Polynucleotide

The polynucleotide preferably has a base sequence encoding the polypeptide described above. In one embodiment, the polynucleotide is preferably in the form of an expression cassette of the polypeptide. The expression cassette is not limited as long as it is a polynucleotide that is capable of expressing a polypeptide in cells. Typical example of the expression cassette is a polynucleotide comprising a promoter and a base sequence that encodes a polypeptide under the control of the promoter.

The promoter contained in the expression cassette is not limited and can be suitably selected according to, for example, the type of cells into which the expression cassette is introduced. Examples include pol II promoters. Specific examples include a CMV promoter, EF1 promoter, SV40 promoter, MSCV promoter, hTERT promoter, β-actin promoter, and CAG promoter. Other examples include a tryptophan promoter, lac promoter, T7 promoter, T5 promoter, T3 promoter, SP6 promoter, arabinose-induced promoter, cold-shock promoter, and tetracycline-induced promoter.

The cell type (origin) used to introduce the polynucleotide and produce the polypeptide is not limited. Examples include animal cells, plant cells, and fungi, such as yeast. Particular examples of animals include various mammals, such as humans, monkeys, mice, rats, dogs, cats, rabbits, pigs, horses, cows, sheep, goats, and deer, non-mammalian vertebrates, and invertebrates. In one embodiment, mammal cells are preferred. The type of cells is not limited, and cells from various tissues or cells having various properties can be used. Examples include blood cells, hematopoietic stem cells/progenitor cells, gametes (sperm and ovum), fibroblast cells, epithelial cells, vascular endothelial cells, nerve cells, liver cells, keratinocytes, muscle cells, epidermal cells, endocrine cells, ES cells, iPS cells, tissue stem cells, and cancer cells.

The expression cassette can comprise other elements (e.g., multiple cloning sites (MCS), drug resistance genes, replication origins, enhancer sequences, repressor sequences, insulator sequences, reporter proteins (e.g., fluorescent protein etc.)-coding sequences and drug resistance gene-coding sequences), if necessary. The MCS is not limited as long as it contains multiple (e.g., 2 to 50, preferably 2 to 20, and more preferably 2 to 10) restriction enzyme sites.

Examples of drug resistance genes include chloramphenicol resistance genes, tetracycline resistance genes, neomycin resistance genes, erythromycin resistance genes, spectinomycin resistance genes, kanamycin resistance genes, hygromycin resistance genes, and puromycin resistance genes.

The reporter protein is not limited as long as it is a light-emitting (color-developing) protein, which emits light (develops a color) by reacting with a specific substrate, or it is a fluorescent protein, which emits fluorescence from excitation light. Examples of light-emitting (color-developing) proteins include luciferase, β-galactosidase, chloramphenicol acetyltransferase, and β-glucuronidase. Examples of fluorescent proteins include GFP, Azami-Green, ZsGreen, GFP2, HyPer, Sirius, BFP, CFP, Turquoise, Cyan, TFP1, YFP, Venus, ZsYellow, Banana, Kusabira-Orange, RFP, DsRed, AsRed, Strawberry, Jred, KillerRed, Cherry, HcRed, and mPlum.

The expression cassette can constitute an expression vector, together with another sequence, if necessary. The type of vector is not limited, and examples include plasmid vectors of animal cell expression plasmids etc.; and virus vectors of retroviruses, lentiviruses, adenoviruses, adeno-associated viruses, herpes viruses, Sendai viruses, etc.

The polynucleotide can be easily prepared according to a known genetic engineering method. For example, the polynucleotide can be prepared using PCR, restriction enzyme cleavage, a DNA ligation technique, etc.

The polynucleotide enables the production of the polypeptide described above. Thus, the polynucleotide can be used for the same purposes as in the polypeptide described above (e.g., a vaccine for diseases or symptoms caused by coronaviruses).

4. Composition

The composition preferably comprises the polypeptide or polynucleotide described above. The composition comprising the polypeptide or polynucleotide described above is useful as a vaccine or a pharmaceutical composition.

The compositions may comprise other components according to the purpose. Examples of other components include a stabilizer for increasing the heat resistance of the vaccine and an adjuvant as an auxiliary for enhancing immunogenicity. Examples of stabilizers include sugars and amino acids. Examples of adjuvants include aluminum compounds (e.g., aluminum hydroxide gel), CpG oligodeoxynucleotide, mineral oil, vegetable oil, alum, bentonite, silica, muramyl dipeptide derivatives, thymosin, and interleukin. The adjuvants may be used alone or in a combination of two or more.

The composition can comprise bases, carriers, solvents, dispersants, emulsifiers, buffers, stabilizers, excipients, binders, disintegrates, lubricants, thickeners, humectants, colorants, fragrances, chelating agents, and the like, if necessary.

Examples of the dosage form of the vaccine or pharmaceutical composition include, but are not limited to, injections, such as aqueous injections, non-aqueous injections, suspension injections, and solid injections; oral preparations, such as tablets, capsules, granules, powders, fine granules, syrups, enteric agents, sustained-release capsules, chewable tablets, drops, pills, liquids for internal use, confectionery tablets, sustained-release preparations, and sustained-release granules; and preparations for external use, such as nasal drops, inhalants, rectal suppositories, inserts, enemas, and jellies.

The content of the active ingredient in the composition can be set based on, for example, the administration subject, the administration route, the dosage form, the conditions of the patient, and the determination of the doctor. For example, the content of the polypeptide or polynucleotide in the composition may be 0.0001 to 95 wt%, and preferably 0.001 to 50 wt%.

The amount of the vaccine or pharmaceutical composition for use can be set in consideration of various factors, such as the administration route, the health conditions of the subject, the age, gender, body weight of the subject, pharmacological findings such as pharmacokinetics and toxicological characteristics, use or non-use of a drug delivery system, whether it is administered as part of a combinational drug with other drugs, acquisition of immunity, and acquisition of boosting effects. For example, the vaccine or pharmaceutical composition can be used such that the administration amount of the active ingredient per single administration is 1 μg to 100 mg/kg (body weight) or 50 μg to 10 mg/kg (body weight). The interval and frequency of administration is not limited, and the administration is preferably performed, for example, at an interval of about 1 to 30 weeks.

5. Antibody and Production Method Therefor

An anti-coronavirus antibody with cross-reactivity can be produced by using the polypeptide described above as an antigen.

Examples of the type of the antibody include polyclonal antibodies, monoclonal antibodies, human chimeric antibodies, humanized antibodies, fully human antibodies, single-stranded antibodies, and antigen-binding fragments (Fab, F(ab')2, minibody, scFv-Fc, Fv, scFv, diabody, triabody, and tetrabody.) A human chimeric antibody is an antibody in which the sequence of the variable region of the antibody is from an animal other than a human (e.g. a mouse or cow) while the sequence of the constant region of the antibody is from a human.

Methods for producing these antibodies are known, and ordinary methods can be used for the production (Current Protocols in Molecular Biology, Chapter 11.12 to 11.13 (2000)). Specifically, for a polyclonal antibody, an antigen can be immunized into a non-human animal, such as a domestic rabbit, to obtain the antibody from the serum of the immunized animal according to an ordinary method. A monoclonal antibody can be obtained from hybridoma cells prepared by immunizing a non-human animal, such as a mouse or a cow, with an antigen, followed by cell fusion of the resulting spleen cells with myeloma cells (Current Protocols in Molecular Biology, edit. Ausubel et al. (1987), publish. John Wiley and Sons, Section 11.4 to 11.11). Immunization of an animal can be conducted by administering the polypeptide described above to an animal or by administering the polynucleotide to the animal and allowing it to express in the animal's body.

The antibody can also be produced by enhancing immunological reactions by using various adjuvants according to the host type. Examples of such adjuvants include mineral gels, such as Freund's adjuvant and aluminum hydroxide; surface-active substances, such as lysolecithin, pluronic polyol, polyanion, peptide, oil emulsion, keyhole limpet hemocyanin, and dinitrophenol; and human adjuvants, such as BCG (Bacille de Calmette et Guérin) and *Corynebacterium parvum.*

In one embodiment, cross-reactivity of an antibody means reactivity to SARS-CoV-2 and coronaviruses other than SARS-CoV-2. In one embodiment, the coronaviruses other than SARS-CoV-2 are those belonging to the genus β-coronavirus, and preferably those belonging to the subgenus Sarbecovirus of the genus β-coronavirus. In one embodiment, the coronaviruses other than SARS-CoV-2 are SARS-related coronaviruses. In one embodiment, examples of the coronaviruses other than SARS-CoV-2 include SARS-CoV-1, WIV1-CoV, MERS-CoV, and Pangolin-CoV. In one embodiment, the antibody preferably recognizes the core-RBD subdomain of the SARS-CoV-2 S protein. In one embodiment, the anti-coronavirus antibodies with cross-reactivity are preferably neutralizing antibodies, and preferably broadly neutralizing antibodies. Such antibodies are useful in preventing, treating, or alleviating diseases or conditions caused by coronaviruses.

Examples

Materials and Test Methods

Mice

BALB/c mice were purchased from SLC Japan and maintained under specific-pathogen-free conditions. Sex-matched 7- to 8-week-old mice were used for all experiments. All animal experiments were conducted in accordance with the animal experiment guidelines of Osaka University.

Human Subjects

Human blood samples were collected at Tokyo Shinagawa Hospital and plasma was isolated using Vacutainer CPT Tubes (Becton, Dickinson). The study protocol was approved by the National Institute of Infectious Diseases Ethic Review Board for Human Subjects, the ethics committees of Tokyo Shinagawa Hospital, and Osaka University. All participants provided written informed consent in accordance with the Declaration of Helsinki.

Vaccine design

The mammalian expression construct for parental rRBD (pCAGGS-CoV-2 RBD-His-Avi) encodes aa 331-530 of SARSCoV-2 S which has MERS S protein-derived signal peptide (MIHSVFLLMFLLTPTESYVD) at the N terminus and a 6×His-Avi-tag (HHHHHHGLNDIFEAQKIEWHE) at the C terminus. For glycan masking, NxT sequons were introduced in the RED surface residues shown in Fig. 2. Mutations expected to disrupt the structure of RBD or with the low glycosylation score (<0.5) by NetNGlyc (http://www.cbs.dtu.dk/ services/NetNGlyc/) were avoided. The candidate constructs were transfected into Expi293F cells to examine whether added glycosylation prevented RBD expression. Antigenicity of the GM mutants was evaluated by ELISA using known anti-RED antibodies.

Recombinant Protein Expression and Purification

The plasmids for the recombinant SARS-CoV RED and GM mutants were transiently transfected into Expi293F cells (Thermo Fisher Scientific) with the ExpiFectamine 293 Transfection Kit (Thermo Fisher Scientific) according to the manufacturer's protocol. After 3-4 days, supernatants were collected and passed through a 0.45-micrometer filter. The recombinant proteins were purified from supernatants by Talon metal affinity resin (Takara) according to the manufacturer's protocol. The elute from the resin was concentrated using an Amicon Ultra 4 10,000 NWML, in which the elution buffer was exchanged to PBS. For nanoparticle antigens and probes, post-translational biotinylation of rRBD and GM mutants was performed in culture via co-expression of the BirA enzyme by co-transfection of a BirA-Flag expression vector (Addgene). Cells were maintained in culture supplemented with 100 μM biotin (Sigma-Aldrich) after transfection as described above.

For preparation of ELISA or ELISPOT antigens, mammalian expression constructs of CoV-2 RBD WT, GM9, GM14 and SARS-related CoV REDs containing a thrombin cleavage site (LVPRGS) in front of the C-terminal 6×His-Avi-tag were transiently transfected into Expi293F cells. Purified proteins were treated with thrombin using a Thrombin kit (Merck) according to the manufacturer's protocol. The cleaved 6×His-AviTag and thrombin were removed by Talon metal affinity resin (Takara) and Benzamidine Sepharose (GE), respectively. Purity or biotinylation efficiency of recombinant proteins was confirmed by sodium dodecyl sulfate-polyacrylamide gel electrophoresis (SDS-PAGE).

Anti-CoV-2 S RBD Monoclonal Antibodies

DNA fragments encoding heavy and kappa or lambda chain variable regions from known anti-CoV-2 mAbs (Pinto et al., 2020, Cross-neutralization of SARS-CoV-2 by a human monoclonal SARS-CoV antibody. Nature 583, 290-295; Yuan et al., 2020, Structural basis of a shared antibody response to SARS-CoV-2. Science 369, 1119-1123; Zhou et al., 2020, Structural basis for the neutralization of SARS-CoV-2 by an antibody from a convalescent patient. Nat Struct Mol Biol 27, 950-958) were synthesized (Eurofins) and cloned into human IgG1, Igkappa and Iglambda expression vectors, respectively. The heavy and light chain expression vectors were co-transfected into Expi293F. Respective mAbs were purified with Protein G Sepharose (GE) according to the manufacturer's protocol.

N-linked Glycan Occupancy Analysis by Liquid Chromatography/Mass Spectrometry (LC/MS)

Sample preparation was performed by referring to Tajiri-Tsukada et al., 2020 (Establishment of a highly precise multi-attribute method for the characterization and quality control of therapeutic monoclonal antibodies. Bioengineered 11, 984-1000). The sample protein (10 μg) was treated using MPEX PTS Reagents (GL Sciences). The reduced and carboxymethylated protein was digested with 2 μg of chymotrypsin (1 ug/ml, Promega) at 37°C for 3 days. The resulting peptides were desalted using an Oasis HLB μElution plate (Waters), and dried and dissolved in 50 μl of 0.1% FA solution. The sample solution was analyzed by liquid chromatography/mass spectrometry (LC/MS) using the parallel acquisition mode using higher-energy collisional dissociation (HCD) and electron-transfer/higher-energy collisional dissociation (EThcD). The EThcD-MS/MS condition was as follows: 23% of supplemental activation collision energy, m/z 2 of isolation window and 100 ms of maximum injection time. The peptide identifications were performed by database searching using BioPharma Finder™ 3.1 (Thermo Fisher Scientific). The search parameters were as follows: a mass tolerance of ±5 ppm, confidence score of >80 and ID type of MS2. Carboxymethylation (+58.005 Da) was set as a static modification of Cys residues. A human glycan database stored in the software was used for glycopeptide identifications. The integrated peak area of the multiple precursor ions from each glycopeptide was calculated, and the relative peak area and glycan occupancy (%) were calculated by the following formula:

$$\text{Relative peak area} = \frac{\text{(The peak area of glycopeptide)}}{\text{(The total peak area of glycopeptides and nonglycosylated peptide)}}$$

$$\text{Glycan occupancy (\%)} = \text{(The total relative peak areas of glycopeptides)/(The total relative peak areas of glycopeptides and nonglycosylated peptide)} \times 100$$

Nanoparticle coating

Streptavidin-coated 0.11-$\mu$m nanoparticles (Bangs Laboratories) were washed twice with PBS and 5.5 $\mu$g nanoparticles were incubated with 50 $\mu$g biotinylated proteins in 80 $\mu$l PBS per one mouse for 5 hours at 4°C. Coating efficiency was measured by flow cytometry.

Immunization

At 7 to 8 weeks of age, each antigen group was vaccinated with a prime immunization, and three weeks later mice were boosted with a second vaccination. Prior to inoculation, immunogen suspensions were gently mixed 2:1 vol/vol with AddaVax adjuvant (InvivoGen) to reach a final concentration of 0.4 mg/mL antigen. Mice were injected intramuscularly using a 29G × 1/2 needle syringe (Terumo) with 60 $\mu$L per injection site (120 $\mu$L total) of immunogen under isoflurane anesthesia. For prime immunization antigens were used as nanoparticles and for boost immunization, antigens were used as monomeric proteins.

Flow cytometry

Single-cell suspensions were prepared from inguinal and iliac lymph nodes or spleen. Inguinal and iliac lymph nodes were collected and pooled for individual mice. Detection of CoV-1 or CoV-2 RBD-specific B cells was carried out using biotinylated rRBD pre-labeled with fluorophore-conjugated streptavidin. To exclude induced 6×His-, AviTag-, biotin- and streptavidin-specific B cells, samples were pre-stained with decoy probe. Cell samples were analyzed using an Attune NxT flow cytometer (Thermo Fisher Scientific) and sorted using a FACS Aria II cell sorter (BD Bioscience). Data were analyzed using FlowJo software V10 (Tree Star).

BCR Cloning and Antibody Expression

CoV-1 and CoV-2-S RBD-specific B cells were single-cell sorted from a lymph node of immunized mice at day 7 after boost as described above. Cloning and expression of antibodies were performed according to a known method (von Boehmer et al., 2016, Sequencing and cloning of antigen-specific antibodies from mouse memory B cells. Nat Protoc 11, 1908-1923; Inoue et al., 2020, Exit from germinal center to become quiescent memory B cells depends on metabolic reprograming and provision of a survival signal. J Exp Med 218). Monoclonal antibodies were expressed using the Expi293 Expression System (Thermo Fisher Scientific) and purified from the culture supernatants of Expi293F cells by Protein G Sepharose (GE).

Flow Cytometry Analysis of mAb Binding to the Antigen

Anti-mouse Igx microparticles and negative control particles (BD CompBeads) were mixed at 1:1 ratio. The beads were then mixed with 250 ng of purified mAbs (mIgG2c/mIgk) for 20 min, washed with PBS containing 2% FBS, followed by labeling with PE-conjugated antigen for 20 min. Binding capacity of mAbs to antigen was assessed by flow cytometry (BD FACSCanto II and FACS Aria II).

ELISA

Nunc Maxisorp Immune plates (Thermo Fisher Scientific) were coated with each rRBD (100 ng/50 $\mu$l). Blocking was carried out using BlockingOne solution (Nacalai Tesque). Plates were sequentially incubated with serially diluted serum samples or monoclonal antibodies cloned from single GC B cells and IgG1-HRP detection antibodies (Southern Biotech). Detection was carried out using KPL SureBlue TMB Microwell Peroxidase Substrate (SeraCare). The CR3022 mouse IgG1e3 monoclonal antibody (InvivoGen) was used as a standard.

For affinity ELISAs, serially diluted sera were incubated on plates coated with 1 $\mu$g/ml CoV-1 RBD protein (low density) or 8 $\mu$g/ml CoV-1 RBD (high density). The affinity of CoV-1 RBD-specific IgG1 was expressed as a ratio of binding to low-density:high-density CoV-1 RED-coated plates as in the disclosure of Wang et al., 2015 (Anti-HA Glycoforms Drive B Cell Affinity Selection and Determine Influenza Vaccine Efficacy. Cell 162, 160-169).

For MAb-based epitope blocking ELISA, CoV-1 RBD or CoV-2 RBD coated plates for serum samples or cloned monoclonal antibodies respectively were prepared. After blocking was carried out, CR3022 (class 4), EY6A (class 4), and/or S309 (class 3) human IgG1 mAbs (150 ng 50 $\mu$l-1 each) in a mixture or alone was added. After incubation at RT for 3 h, the plate was washed with PBST, then serum samples or cloned monoclonal antibodies were added and incubated at 37°C for 1 h. After three washes, HRP conjugated goat anti-mouse IgG1 or IgG2c, respectively, were added and incubated for 1 h at RT. Occupancy of Class 3/4 type antibodies in whole anti-CoV-1 RBD antibodies was calculated by subtraction of titers measured by epitope blocking ELISA and conventional serum titer ELISA and then showed as a percentage.

ELISPOT Assay

Plates with a cellulose membrane bottom were coated with CoV-1 RBD or CoV-2 RBD (100 ng 50 $\mu$l-1). Bone marrow cells were added to the wells and incubated in RPMI1640 medium supplemented with 10% FBS, 50uM 2-ME and 2 mM sodium pyruvate for 5 h at 37°C under 5% $CO_2$. After washing with PBS with 0.05% Tween 20, goat anti-mouse IgG1 Ab was added to the wells followed by addition of alkaline phosphatase-labeled anti-goat IgG Ab. Spots were visualized by BCIP/NBT substrate (Promega) and counted.

Pseudovirus Assay

Preparation of SARS CoV S protein-pseudotyped VSV$\Delta$G-luc was performed according to the disclosures of Tani et al., 2010 (Involvement of ceramide in the propagation of Japanese encephalitis virus. J Virol 84, 2798-2807) and Yoshida et al., 2021 (SARS-CoV-2-induced humoral immunity through B cell epitope analysis in COVID-19 infected individuals. Sci Rep 11, 5934). HEK293T cells were transfected with expression plasmids for respective CoV S proteins (SARS-CoV-2 Wuhan strain S, SARS-CoV-1 S, and SARS-related CoV WIV1 S) by using TransIT-LT1 (Mirus) according to the manufacturer's instructions. At 24 hours after transfection, VSV$\Delta$G-luc virus (MOI = 0.1) was inoculated onto the transfectants. After 2-hour incubation, cells were washed with DMEM and were further cultivated for additional 24-48 hours. Cell-free supernatant was harvested and used for the neutralizing assay according to the disclosure of Nie et al., 2020 (Quantification of SARS-CoV-2 neutralizing antibody by a pseudotyped virus-based assay. Nat Protoc 15, 3699-3715). Mouse sera and human plasma samples were incubated at 56°C for 30 min and then serially diluted from 1/20 in culture medium. SARS-CoV S-pseudotyped VSV$\Delta$G-luc was incubated with different dilution of mouse sera, human plasma or recombinant antibodies for 1 hour at 4°C, and then inoculated onto a monolayer culture of VeroE6-TMPRSS2 in a 96-well plate. At 16-hour post inoculation, cells were washed with PBS and then lysed with luciferase cell culture lysis reagent (Promega). After centrifugation, cleared cell lysates were incubated with firefly luciferase assay substrate (Promega) in 96-well white polystyrene plates (Corning). Luciferase activity was measured by GloMax Discover luminometer (Promega). NT50 or $IC_{50}$ was calculated by Prism software (GraphPad).

Antibody-dependent Enhancement (ADE) Assay

DENV1 single-round infectious particles (SRIP) were prepared according to the disclosure in Matsuda et al., 2018 (High-throughput neutralization assay for multiple flaviviruses based on single-round infectious particles using dengue virus type 1 reporter replicon. Sci Rep 8, 16624). For evaluation of the ADE assay system using RAW264.7 cells, anti-E mouse monoclonal antibody 3H12 (Yamanaka et al., 2013, A mouse monoclonal antibody against dengue virus type 1 Mochizuki strain targeting envelope protein domain II and displaying strongly neutralizing but not enhancing activity. J Virol 87, 12828-12837) was pre-incubated with DENV1 SRIP (multiplicity of infection [MOI] = 0.1) for 1 h at 4°C, and then inoculated onto RAW264.7 cells in 96-well plate. After 2 hours, cells were washed with PBS and cultivated in fresh medium for 3 days. Infectivity was measured using a NanoLuc assay kit (Promega). To examine whether antisera had ADE of CoV-2 infection, SARS-CoV-2 S pseudotyped VSV$\Delta$G-luc was incubated with sera from immunized mice for 1 h at 4°C, and then inoculated onto RAW264.7 cells in a 96-well plate. After 2 hours, cells were washed with PBS and cultivated in fresh medium for 16-20 hours. Luciferase activity of infected cells was measured as above.

Virus Neutralization Assay

A mixture of 100 TCID50 virus and serially diluted, heat-inactivated plasma samples (2-fold serial dilutions starting from 1:40 dilution) were incubated at 37°C for 1 h before being placed on VeroE6-TMPRSS2 cells seeded in 96-well flat-bottom plates (TPP). VeroE6-TMPRSS2 cells were maintained in low glucose DMEM (Fujifilm) containing 10% heat-inactivated fetal bovine serum, 1 mg/mL Geneticin (Thermo Fisher Scientific), and 100 U/mL penicillin/streptomycin (Thermo Fisher Scientific) at 37°C supplied with 5% $CO_2$. After culturing for 4 days, cells were fixed with 20% formalin (Fujifilm), and stained with crystal violet solution (Sigma-Aldrich). Cut-off dilution index with >50% cytopathic effect was presented as microneutralization titer. Microneutralization titer of the sample below the detection limit (1:40 dilution) was set as 20.

Biolayer Interferometry Assay

The kinetics of monoclonal antibody binding to antigen was determined with the OctetRED96e system (ForteBio) at 30°C with shaking at 1,000 rpm. Biotinylated-RED proteins from CoV-1, CoV-2, WIV1-CoV, Bat SARS-related AL-103 and BM48-31 were loaded at 6 ug/ml in 1× kinetics buffer (0.1% BSA, 0.02% Tween-20 in PBS) for 900 s onto streptavidin biosensors (ForteBio) and incubated with serially diluted Fab antibodies (100, 33.3, 11.1, and 0 nM) for 120 s, followed by immersion in 1× kinetics buffer for 300 s of dissociation time. The binding curves were fit in a 1:1 binding model and the dissociation constant (KD) values were calculated by Octet Data Analysis software (ForteBio).

Phylogenetic Analysis of Antibody Clones

For each cell, V,D,J gene and CDR3 assignment was conducted on the full length BCR sequences using IgBlast and the IMGT mouse references. Clones were defined separately for each experiment, on the basis of their heavy chain only, using the DefineClones function of the Change-O package, and 38/38 cells of GM14#1 and 26/31 cells of GM14#2 were considered the same clone. Clonal tree reconstruction was performed for these two major clones using Alakazam (both Change-O and Alakazam are part of the Immcantation analysis framework (Gupta et al., 2015, Change-O: a toolkit for analyzing large-scale B cell immunoglobulin repertoire sequencing data. Bioinformatics 31, 3356-3358)). Reconstructed clonal trees were finally plotted using Cytoscape.

Statistics

Statistical analyses were performed by a two-tailed unpaired and paired Student's t-test using GraphPad Prism software. P values <0.05 were considered significant. Error bars denote ± SD.

Design of SARS-CoV-2 RBD glycan mutants

To engineer the SARS-CoV-2 head-RBD subdomain (Fig. 1A), N-linked glycosylation sites (NxT motif) were introduced in this subdomain (Barnes et al., 2020, Structures of Human Antibodies Bound to SARS-CoV-2 Spike Reveal Common Epitopes and Recurrent Features of Antibodies. Cell 182, 828-842 e816), which contains essential residues for ACE-2 engagement (Fig. 1A and Fig. 1C). The already established monoclonal antibodies (mAbs) recognizing SARS-CoV-2 RBD are classified into four types; as shown in Fig. 1B, class 1/2 and 3/4 recognize epitopes in the head-RBD and core-RBD subdomain, respectively. Five potential sites for the introduction of NxT sequons (GM14) were identified based on the following criteria; (1) surface residues on the class 1 or 2 epitope; (2) residues outside the class 3 and 4 epitopes in the core-RBD subdomain (Barnes et al., 2020, the same as above) (Fig. 1C); (3) residues within, or conformationally close to the non-conserved patches of the head-RBD subdomain (A in Fig. 2); (4) NxT mutations not expected to disrupt the overall structure of RBD; (5) not expected to reduce RBD protein expression or stability (Starr et al., 2020, Deep Mutational Scanning of SARS-CoV-2 Receptor Binding Domain Reveals Constraints on Folding and ACE2 Binding. Cell 182, 1295-1310 e1220). Although the N415 site is somewhat distal from the head-RBD subdomain (Fig. 1C), this site was already reported to be a part of the epitope recognized by the class 1 mAb CB6 (Shi et al., 2020, A human neutralizing antibody targets the receptor-binding site of SARS-CoV-2. Nature 584, 120-124). Two glycan mutants GM9 and GM14 that have 3 and 5 additional glycosylation sites, respectively, were designed. Structural modeling predicted that these additional glycans would prevent antigen recognition by class 1 and 2 antibodies (Barnes et al., 2020, the same as above; Yuan et al., 2020, the same as above).

Results

The glycan mutants were coupled with 6×His-Avi-tag at the C terminus and expressed in mammalian Expi293 cells. They exhibited a higher molecular weight than wildtype RBD in SDS-PAGE (Fig. 2B). The glycan occupancy of each introduced glycosylation sites was determined by liquid chromatography-mass spectrometry (LC-MS). All added glycosylation sites had occupancies greater than 75% except the added N494 and N502 glycan from GM9, which showed 70% and 57%, respectively (Fig. 1D). The process of N-glycosylation in the endoplasmic reticulum (ER) is affected by conformational constraints (Breitling and Aebi, 2013, N-linked protein glycosylation in the endoplasmic reticulum. Cold Spring Harb Perspect Biol 5, a013359). The somewhat lower glycan occupancy at N494 and N502 could be due to limited accessibility of oligosaccharyl transferases to these asparagine residues, which are positioned in close proximity to each other.

It was then investigated whether the glycan modifications prevent binding of class 1/2 antibodies but maintain antigenic epitopes for class 3/4 antibodies. In ELISA, all tested antibodies were strong binders to wildtype RBD (Fig. 1E). mAb CB6, a stereotypic class 1 neutralizing antibody, completely failed to bind to either of the glycan mutants (GM9 and GM14). Although the binding of mAb C002, a class 2 antibody (Robbiani et al., 2020, Convergent antibody responses to SARS-CoV-2 in convalescent individuals. Nature 584, 437-442), to RED was abolished by GM14, this mAb still bound weakly to GM9, indicating that the five mutations are required for complete inhibition of class 2 mAb C002. In contrast, core-RBD subdomain targeting mAb S309 (class 3), mAb CR3022 (class 4), and mAb EY6A (class 4) (Pinto et al., 2020, the same as above; Yuan et al., 2020, the same as above) (Zhou et al., 2020, the same as above) bound to wildtype RBD, GM9, or GM14 at a similar level, indicating that the introduced mutations for additional glycosylation do not affect overall structure of the core-RBD subdomain (Fig. 1E).

Glycan engineering of the SARS-CoV-2 head-RED generated more antibodies towards the core-RBD with higher affinity

To overcome the limited immunogenicity of the relatively small SARS-CoV-2 RBD, it was multivalently displayed on streptavidin polystyrene nanoparticles. Consistent with the disclosure in Walls et al., 2020 (Elicitation of Potent Neutralizing Antibody Responses by Designed Protein Nanoparticle Vaccines for SARS-CoV-2. Cell 183, 1367-1382 e1317), in contrast to soluble monomeric RBD, the particulate RBD gave rise to more robust primary antibody responses (Fig. 4). These particulate antigens (wildtype RBD, GM9, or GM14) were injected intramuscularly into BALB/c mice with AddaVax adjuvant. The mice were boosted with respective non-particulate antigens 3 weeks after priming (Fig. 3A). Sera taken at 7 days after boosting were tested for reactivity to SARS-CoV-2 RBD. Since, like alum, AddaVax dominantly induces IgG1 type antibody in the mouse (Sato et al., 2020, Repurposing the psoriasis drug Oxarol to an ointment adjuvant for the influenza vaccine. Int Immunol 32, 499-507), the IgG1 response was measured. To first address the possibility that the mice may have mounted an immune response towards neo-epitopes generated by glycan engineering that the parental SARS-CoV-2 RBD probe would not detect, serum and GC B cell responses were examined from the respective immunization protocols using immunogen-matched glycosylation mutant and parental probes. ELISA assay analysis indicated similar RBD-specific serum titers between the probe sets; by flow cytometry analysis, about 10 or 15% of GC B cells bound to only immunogen-matched glycosylation mutant probes (Figs. 6A and B). Hence, to some extent, B cells recognizing neo-epitopes appear to emerge upon GM9 or GM14 immunization. Nevertheless, more than 85% of the responding cells were reactive to the parental CoV-2 RBD probe, thus, the parental SARS-CoV-2 RBD probe were usually used in these assays.

The IgG1 from mice immunized with glycan mutants displayed similar reactivity to CoV-2, assessed by ELISA, to that by wildtype RBD immunization (Fig. 3B, left). In regard to the reactivity to CoV-1, GM9- or GM14-immunized mice exhibited about 8-fold higher than wildtype RBD immunization (Fig. 3B, right). Given the sequence conservation of the RBD-core subdomain between SARS-CoV-2 and CoV-1 (Fig. 2A), it is most likely that the enhanced cross-reactivity to CoV-1 by GM9 or GM14 immunization is due to generation of more IgG1 towards the conserved RBD-core subdomain of CoV-2. Supporting this possibility, CoV-1 reactive IgG1 elicited by GM9 or GM14 immunization was about 90 or 85% inhibited by pre-treatment of verified class 3/4 anti-CoV-2 core-RED antibodies (mixtures of mAb S309, mAb CR3022, and mAb EY6A), respectively, like wildtype RBD immunization (Fig. 3C, left). Next, the affinity of cross-reactive IgG1 elicited by GM9 or GM14 immunization was characterized. The affinity of IgG1 for CoV-1 RED was measured using an affinity ELISA assay that was modified from the nitrophenyl system; this assay measures the ratio of high-affinity to all-affinity binding IgG1. As shown in Fig. 3C, right, in contrast to wildtype RBD immunization, cross-reactive IgG1 elicited by GM9 or GM14 immunization had significantly higher affinity. When the WIV1-CoV was used instead of the SARS-CoV-1 RBD probe, both glycan mutants induced about 8-fold higher serum titers (Fig. 3D, left). In regard to affinity, although two among 11 wildtype RED-immunized mice showed high ratio of high-affinity to all-affinity binding, sera from the remaining nine mice had relatively lower affinity compared with GM9- or GM14-immunized mice (Fig. 3D, right). Together, glycan engineering of the SARS-CoV-2 head-RBD subdomain facilitates the generation of core-RBD subdomain specific antibodies with higher affinity.

Glycan engineering of the SARS-CoV-2 head-RED elicited cross-neutralizing antibodies against SARS-CoV-1 and other related viruses

To determine neutralizing activity, a vesicular stomatitis virus (VSV)-based pseudovirus method (Nie et al., 2020, the same as above) was mainly employed, and half-maximal neutralization titers (NT50s) were calculated. CoV-2 wildtype RED, GM9, or GM14 immunization gave rise to similar neutralizing activity to SARS-CoV-2. GM9 or GM14 immunization manifested somewhat increased or decreased activity, respectively, compared with CoV-2 wildtype RBD immunization (Fig. 5A). By using virus neutralizing assay, this tendency by GM9 or GM14 immunization was also observed (Fig. 8).

To measure the neutralizing activity against CoV-2 variants, VSV-based viruses with K417N/E484K/N501Y mutation from the B.1.351 variant were used. The results indicated that even CoV-2 wildtype RBD immunization elicited somewhat higher neutralizing activity than wildtype CoV-2. Given that human sera by mRNA vaccines showed low neutralizing activity against viruses with the E484K mutation (Chen et al., 2021, Resistance of SARS-CoV-2 variants to neutralization by monoclonal and serum-derived polyclonal antibodies. Nat. Med. 27:717-726), the immunization technique of the present invention can produce more resistant sera.

Towards SARS-CoV-1, antisera elicited by GM9 or GM14 immunization possessed neutralizing activity with about 15- or 10-fold higher potency, respectively, than CoV-2 wildtype RBD immunization, assessed by the VSV-based pseudovirus method (Fig. 5A). Similarly, these sera exhibited substantial neutralizing activity against WIV1-CoV, SHC014, and PaGX (Fig. 5A). Importantly, the levels of neutralization for SARS-CoV-1 and WIV1-CoV observed after GM9 or GM14 immunization were similar to convalescent plasma samples from SARS-CoV-2-infected individuals for their neutralizing activity of the SARS-CoV-2, analyzed by the same laboratory in the same assay format (Fig. 5B and Fig. 5C). Given that the major epitopes of the CoV-1 reactive antibodies elicited by GM9 or GM14 immunization are located in the core-RBD subdomain (Fig. 3C, left), it is most likely that these anti-core-RBD antibodies contribute to cross-neutralization of SARS-CoV-1, WIV1-CoV, SHC014, and PaGX.

Previous studies of SARS-CoV-1 and other viruses have suggested that antibody-dependent enhancement (ADE) of infection might take place after vaccination (Smatti et al., 2018, Viral-Induced Enhanced Disease Illness. Front Microbiol 9, 2991). To examine this possibility, VSV-based pseudovirus was incubated with serially diluted sera from immunized mice and then inoculated on Raji, a human B lymphoma cell line frequently used to evaluate the ADE activity of SARS-CoV (Verma et al., 2009, Analysis of the Fc gamma receptor-dependent component of neutralization measured by anthrax toxin neutralization assays. Clin Vaccine Immunol 16, 1405-1412). As a positive control for this assay, it was shown that infectivity of CoV-2 S pseudovirus was significantly enhanced by addition of anti-CoV-2 SRBD MW05 chimeric human/mouse IgG1 antibody (Fig. 5D, center). In this experimental setting, like sera from mice immunized with SARS-CoV-2 wildtype RBD, sera from mice immunized with GM9 or GM14 showed no significant ADE activity (Fig. 5D, right).

Reactivity profiles of cross-reactive GC B cells

To complement the above serological characterization, the reactivity of GC B cells isolated post priming was assessed. As demonstrated by flow analysis, wildtype RED, GM9, or GM14 immunization gave rise to similar levels of overall GC B cells (Fig. 7A). Among the CoV-2+ cells, the frequency of GC B cells positive for both CoV-2 and CoV-1 probes were increased by immunization of GM9 or GM14, compared with wildtype RBD immunization (Figs. 7B and C).

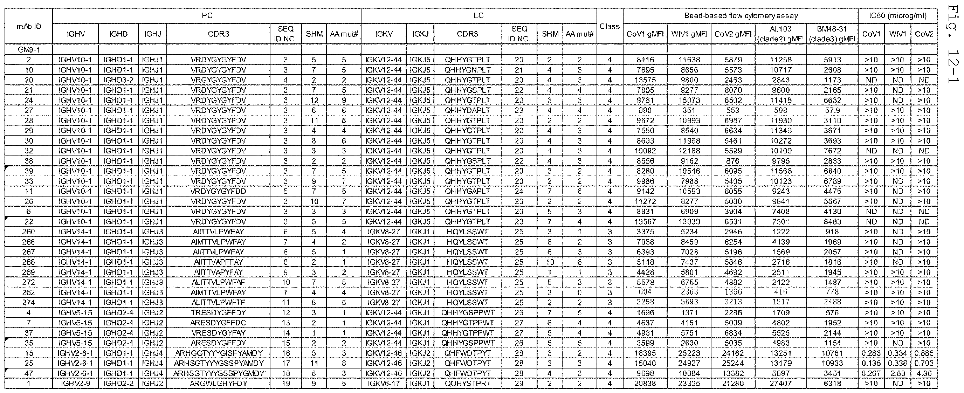

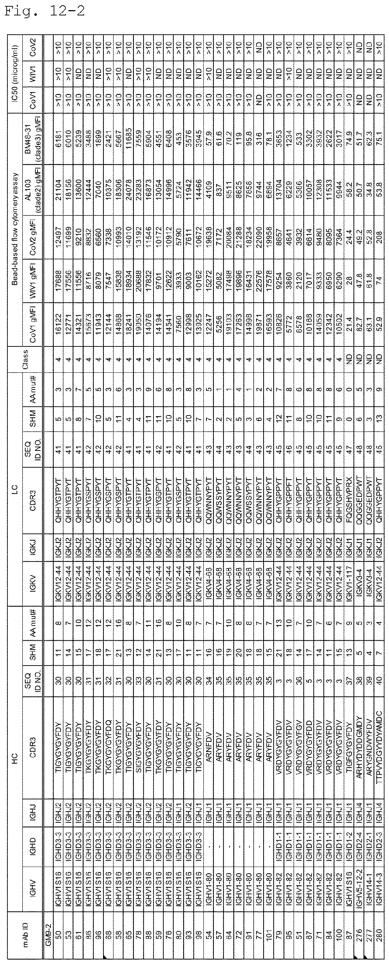

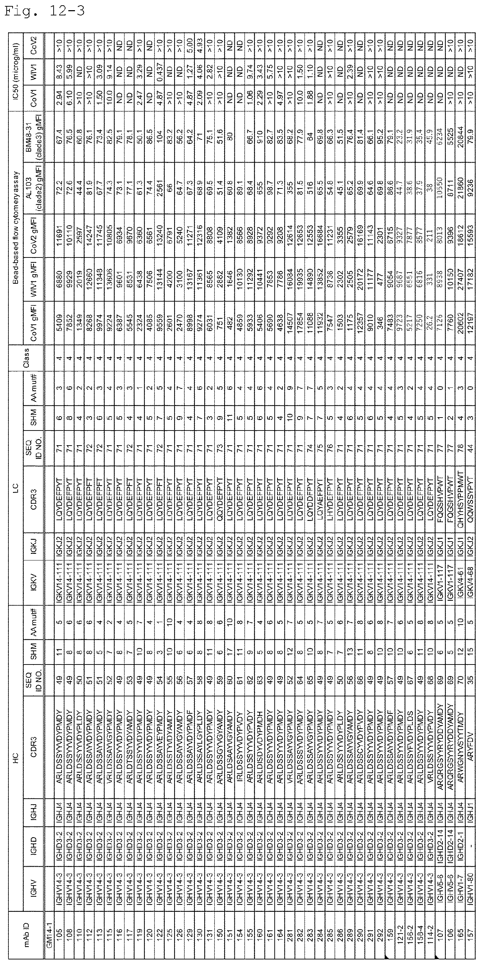

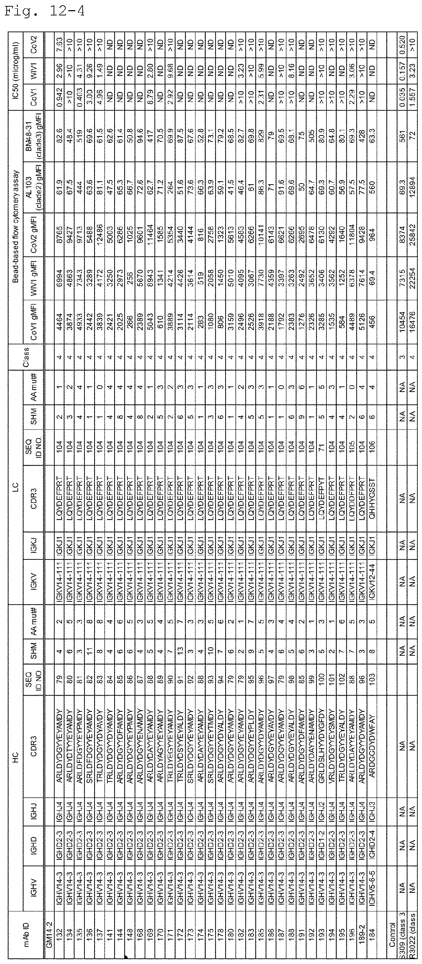

To examine cross-reactivity profiles for each CoV-2$^+$/CoV-1$^+$ GC B cells, mAbs from single cell sorted GC B cells from four individual mouse (GM9-1, GM9-2, GM14-1, or GM14-2) were characterized. Sequence analysis revealed that, in each mouse, more than 85% of the isolated mAbs were members of expanded clonal lineages. Particularly in GM14-1 or GM14-2 mouse, almost all the mAbs were encoded by the same combination of VH/VK with different DH and JK segments: GM14-1 (VH14-3-DH3-2-JH4/VK4-111-JK2), and GM14-2 (VH14-3-DH2-3-JH4/VK4-111-JK1) (Fig. 12).

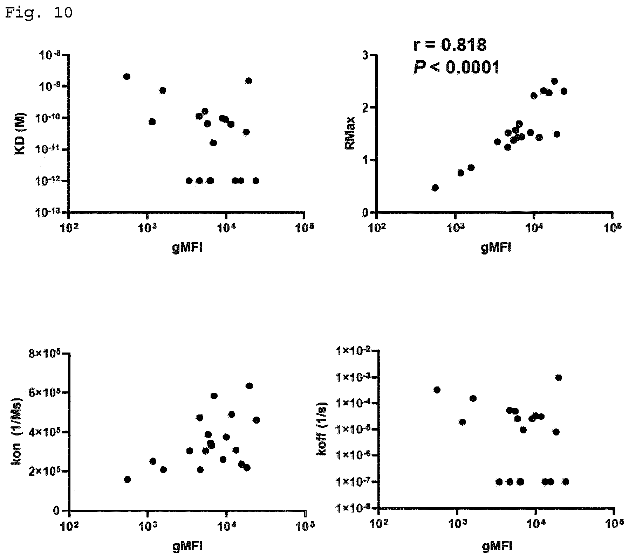

Among all the analyzed mAbs, many of them showed blockade of their binding to CoV-2 RBD by authentic class 4 mAbs, except that mAbs derived from VH14-1/VK8-27 gene pairing were blocked by authentic class 3 mAb (Fig. 12). For assessment of the breath of recognition to sarbecovirus REDs by the above mAbs, a relative binding index was assigned to each antibody by employing flow cytometry-based measurement (Fig. 9A) (Bajic et al., 2019, Influenza Antigen Engineering Focuses Immune Responses to a Subdominant but Broadly Protective Viral Epitope. Cell Host Microbe 25, 827-835 e826; Leach et al., 2019, Requirement for memory B-cell activation in protection from heterologous influenza virus reinfection. Int Immunol 31, 771-779); the relative binding indexes represented well RMax of bivalent IgG antibodies measured by Octet kinetics assay (Fig. 10). RBDs were selected from distantly related clade 2 (Anlong-103 [AL-103]) and clade 3 (BM48-31) (Lau et al., 2020, Possible Bat Origin of Severe Acute Respiratory Syndrome Coronavirus 2. Emerg Infect Dis 26, 1542-1547) (Letko et al., 2020, Functional assessment of cell entry and receptor usage for SARS-CoV-2 and other lineage B betacoronaviruses. Nat Microbiol 5, 562-569), in addition to clade 1 (SARS-CoV-1 and WIV1-CoV as clade 1a, and SARS-CoV-2 and PaGX as clade 1b). As expected, almost all the isolated mAbs from GM9-1, GM9-2, GM14-1, or GM14-2 mouse displayed binding reactivity to clade 1a and clade 1b REDs. The relative binding indexes were well correlated between CoV-1/WIV1/SHC014/PaGX and CoV-2 in GM9- or GM14-immunized mice (Fig. 9B). In regard to clade 2 and 3RBD, some of the isolated mAbs from GM9-1 or GM9-2, but only a few from GM14-1 mouse, exhibited their significant binding reactivity (Fig. 9A).

Then, the neutralization activities of each mAb towards CoV-1, WIV1, SHC014, PaGX, or CoV-2 were evaluated by the aforementioned VSV-based pseudotype assays (Fig. 9A and Fig. 12). Because of the large number of antibodies, screening was performed and nine mAbs were focused using the following two criteria (Fig. 9C). First, the neutralization activities are below 10 $\mu$g/ml IC$_{50}$ (half-maximal inhibitory concentration) towards at least two types of viruses among three. The highest potency towards these viruses is below 3 $\mu$g/ml IC$_{50}$. As shown in Fig. 9D, like authentic mAb S309 or CR3022, these mAbs possessed higher IC$_{50}$s for CoV-2 than those observed for CoV-1 or WIV1. In VH14-3-DH3-2-JH4/VK4-111-JK2 (GM14-1) or VH14-3-DH2-3-JH4/VK4-111-JK1 (GM14-2) lineage, it was demonstrated that the mAb with the highest binding index for CoV-2 does not possess the highest potency of neutralization activity towards CoV-2 (Fig. 9C). Thus, antibody neutralization potency is likely governed at least in part by factors beyond binding affinity.

Elicitation of cross-reactive long-lived plasma cells and memory B cells

Most successful vaccine approaches rely on the generation of memory B cells and long-lived plasma cells (LLPCs). As shown in Fig. 11A, analysis was carried out at seven weeks. At this time, class-switched IgG1 type memory B cells were similarly generated by wildtype RBD, GM9, or GM14 immunization, although GM9 and GM14 gave rise to more CoV-1+CoV-2+ cells (Fig. 11B). Furthermore, as assessed by SARS-CoV-1-specific antibody secreting cells (ASC) in the bone marrow, GM9 or GM14 immunization induced more CoV-1+ ASC (Fig. 11C), together indicating that both cross-reactive memory compartments are more efficiently generated by this immunization protocol using GM9 or GM14, compared with wildtype RBD immunization.

**Claims**

1. A polypeptide comprising an amino acid sequence having at least 60% or more identity to the amino acid sequence of SEQ ID NO: 2, wherein the amino acid sequence is modified so as to allow sugar chain attachment to positions corresponding to positions 85, 164, and 172 of the amino acid sequence of SEQ ID NO: 2.

2. The polypeptide according to claim 1, wherein the modified amino acid sequence is further modified so as to allow sugar chain attachment to a position corresponding to position 118 and/or a position corresponding to position 147 of the amino acid sequence of SEQ ID NO: 2.

3. The polypeptide according to claim 1 or 2, wherein the sugar chain is an N-type sugar chain.

4. The polypeptide according to claim 1 or 2, wherein a sugar chain is attached to each of positions corresponding to positions 85, 164, and 172 of the amino acid sequence of SEQ ID NO: 2.

5. The polypeptide according to claim 4, wherein a sugar chain is further attached to a position corresponding to position 118 and/or a position corresponding to position 147.

6. The polypeptide according to claim 4, wherein the sugar chain is an N-type sugar chain.

7. A polynucleotide comprising a base sequence encoding the polypeptide of claim 1.

8. A composition comprising the polypeptide of any one of claims 1 to 6 or the polynucleotide of claim 7.

9. The composition according to claim 8, which is a vaccine.

10. A method for producing an anti-coronavirus antibody, comprising immunizing an animal with the polypeptide of any one of claims 1 to 6.

11. An antibody obtained by the method of claim 10.

Fig. 1-1

A

B

Side view                    Top view

Fig. 1-2

Fig. 2-1

A

Side view          Top view

RBD { Head, Core

B

|  | Added glycans | | | | |
| AA number | 415 | 448 | 477 | 494 | 502 |
|---|---|---|---|---|---|
| WT | TGK | NYN | STP | SYG | GVG |
| GM9 | NGT | | | NYT | NVT |
| GM14 | NGT | NYT | NTT | NYT | NVT |

Fig. 2-2

Fig. 3-1

A
Week0    Week3    Week4

Immunization;
25µg X2, with Addavax

Immunization;
25µg X2, with Addavax

Blood collected

B

Anti-CoV-2 RBD

Anti-CoV-1 RBD

Fig. 3-2

C

D

Anti WIV1 S RBD

Fig. 4

Fig. 5-1

Fig. 5-2

## C

|  | Healthy control | Convalescent patients |
|---|---|---|
| n | 8 | 24 |
| Mean age | 33.8 (22-48) | 50.7 (22-93) |
| Sex | 5 M/3 F | 14 M/10 F |
| Time after onset | N/A | 58.2 days (20-115) |
| Severity | N/A | Severe: 3 Moderate: 21 |

D

Fig. 6

A

B

Fig. 7

Fig. 8

Fig. 9-1

Fig. 9-2

EP 4 328 236 A1

Fig. 9-3

Fig. 9-4

Fig. 9-5

**B**

GM9

GM14

**C**

|  | Affinity ($K_D$ [M]) | | | | | | | | | Neutralization ($IC50$ [µg/ml]) | | | | |
|---|---|---|---|---|---|---|---|---|---|---|---|---|---|---|
|  | Clade 1a | | | 1b | | 2 | | | 3 | Clade 1a | | | 1b | |
|  | CoV-1 | WIV1 | SHC014 | CoV-2 | PaGX | AL-103 | Rs4048 | Rf1/2004 | BM48-31 | CoV-1 | WIV1 | SHC014 | CoV-2 | PaGX |
| Ab 15 | 1.90E-09 | 1.66E-09 | 2.31E-09 | 1.21E-09 | 5.43E-09 | 4.43E-09 | 7.94E-09 | 7.95E-08 | 7.31E-08 | 0.283 | 0.334 | 0.471 | 0.885 | 1.40 |
| Ab 25 | 9.21E-09 | 7.55E-09 | 5.15E-09 | 4.54E-09 | 8.29E-09 | 8.01E-09 | 1.47E-08 | 8.21E-07 | 1.29E-06 | 0.135 | 0.338 | 0.181 | 0.703 | 0.292 |
| Ab 47 | 2.50E-08 | 4.02E-08 | 2.47E-08 | 2.10E-08 | 3.56E-08 | 6.30E-08 | 1.92E-07 | nb | nb | 0.367 | 2.83 | 1.47 | 4.38 | 3.77 |
| Ab 129 | 1.46E-08 | 2.07E-09 | 1.06E-06 | | 2.38E-09 | nb | nb | nb | nb | 4.87 | 1.27 | >10 | 5.00 | 3.43 |
| Ab 130 | 2.67E-08 | 1.06E-08 | 5.09E-07 | 4.86E-09 | 1.43E-08 | nb | nb | nb | nb | 2.09 | 4.06 | >10 | 4.93 | 1.41 |
| Ab 283 | 2.00E-08 | 7.20E-08 | 3.07E-06 | 2.55E-09 | 5.37E-09 | nb | nb | nb | nb | 1.88 | 1.10 | >10 | >10 | >10 |
| Ab 132 | 2.37E-08 | 1.35E-08 | 5.30E-07 | | 2.38E-09 | nb | nb | nb | nb | 0.942 | 2.96 | >10 | 7.83 | >10 |
| Ab 137 | 1.84E-08 | 2.03E-09 | 4.81E-07 | 2.26E-09 | 5.31E-09 | nb | nb | nb | nb | 4.96 | 1.49 | >10 | >10 | >10 |
| Ab 185 | 2.76E-08 | 1.96E-08 | 5.10E-07 | 7.69E-09 | 2.48E-08 | nb | nb | nb | nb | 2.31 | 5.99 | >10 | >10 | >10 |

Fig. 9-6

Fig. 10

Fig. 11

**A**

Week0      Week3      Week7

Immunization;    Immunization;    Flow cytometry and
25 µg X2, with Addavax   25 µg X2, with Addavax   ELISPOT

**B**

CoV-2 wt      GM9      GM14

27.8    54.3    54.5

CoV-2 RBD    CoV-1 RBD

**C**

Fig. 12-1

| mAb ID | HC | | | | | | | LC | | | | | | Class | Bead-based flow cytomery assay | | | | | IC50 (microg/ml) | | |
|---|---|---|---|---|---|---|---|---|---|---|---|---|---|---|---|---|---|---|---|---|---|---|
| | IGHV | IGHD | IGHJ | CDR3 | SEQ ID NO. | SHM | AA mut# | IGKV | IGKJ | CDR3 | SEQ ID NO. | SHM | AA mut# | | CoV1 gMFI | WIV1 gMFI | CoV2 gMFI | AL103 (clade2) gMFI | BM48-31 (clade3) gMFI | CoV1 | WIV1 | CoV2 |
| GM9-1 | | | | | | | | | | | | | | | | | | | | | | |
| 2 | IGHV10-1 | IGHD1-1 | IGHJ1 | VRDYGYGYFDV | 3 | 5 | 5 | IGKV12-44 | IGKJ5 | QHHYGTPLT | 20 | 2 | 2 | 4 | 8416 | 11638 | 5879 | 11258 | 5913 | >10 | >10 | >10 |
| 10 | IGHV10-1 | IGHD1-1 | IGHJ1 | VRDYGYGYFDV | 3 | 7 | 5 | IGKV12-44 | IGKJ5 | QHHYGNPLT | 21 | 4 | 3 | 4 | 7695 | 8656 | 5573 | 10717 | 2608 | >10 | >10 | >10 |
| 20 | IGHV10-1 | IGHD3-2 | IGHJ1 | VRGYGYGYFDV | 4 | 2 | 2 | IGKV12-44 | IGKJ5 | QHHYGTPLT | 20 | 4 | 3 | 4 | 13575 | 9800 | 2463 | 2843 | 1173 | ND | ND | ND |
| 21 | IGHV10-1 | IGHD1-1 | IGHJ1 | VRDYGYGYFDV | 3 | 7 | 5 | IGKV12-44 | IGKJ5 | QHHYGSPLT | 22 | 4 | 4 | 4 | 7805 | 9277 | 6070 | 9600 | 2165 | >10 | ND | >10 |
| 24 | IGHV10-1 | IGHD1-1 | IGHJ1 | VRDYGYGYFDV | 3 | 12 | 9 | IGKV12-44 | IGKJ5 | QHHYGTPLT | 20 | 3 | 3 | 4 | 9761 | 15073 | 6502 | 11418 | 6632 | >10 | ND | >10 |
| 27 | IGHV10-1 | IGHD1-1 | IGHJ1 | VRDYGYGYFDV | 3 | 6 | 6 | IGKV12-44 | IGKJ5 | QHHYDAPLT | 23 | 4 | 4 | 4 | 990 | 351 | 553 | 598 | 57.9 | >10 | >10 | >10 |
| 28 | IGHV10-1 | IGHD1-1 | IGHJ1 | VRDYGYGYFDV | 3 | 11 | 8 | IGKV12-44 | IGKJ5 | QHHYGTPLT | 20 | 2 | 2 | 4 | 9672 | 10993 | 6957 | 11930 | 3110 | >10 | >10 | >10 |
| 29 | IGHV10-1 | IGHD1-1 | IGHJ1 | VRDYGYGYFDV | 3 | 4 | 4 | IGKV12-44 | IGKJ5 | QHHYGTPLT | 20 | 4 | 3 | 4 | 7550 | 8540 | 6634 | 11349 | 3671 | >10 | >10 | >10 |
| 30 | IGHV10-1 | IGHD1-1 | IGHJ1 | VRDYGYGYFDV | 3 | 8 | 6 | IGKV12-44 | IGKJ5 | QHHYGTPLT | 20 | 4 | 3 | 4 | 8603 | 11968 | 5461 | 10272 | 3693 | >10 | >10 | >10 |
| 32 | IGHV10-1 | IGHD1-1 | IGHJ1 | VRDYGYGYFDV | 3 | 3 | 3 | IGKV12-44 | IGKJ5 | QHHYGTPLT | 20 | 4 | 3 | 4 | 10092 | 12188 | 5599 | 10100 | 7672 | ND | ND | ND |
| 38 | IGHV10-1 | IGHD1-1 | IGHJ1 | VRDYGYGYFDV | 3 | 2 | 2 | IGKV12-44 | IGKJ5 | QHHYGSPLT | 22 | 4 | 3 | 4 | 8556 | 9162 | 876 | 9795 | 2833 | >10 | >10 | >10 |
| 39 | IGHV10-1 | IGHD1-1 | IGHJ1 | VRDYGYGYFDV | 3 | 7 | 5 | IGKV12-44 | IGKJ5 | QHHYGTPLT | 20 | 3 | 2 | 4 | 8280 | 10546 | 6095 | 11566 | 6840 | >10 | >10 | >10 |
| 33 | IGHV10-1 | IGHD1-1 | IGHJ1 | VRDYGYGYFDV | 3 | 9 | 7 | IGKV12-44 | IGKJ5 | QHHYGTPLT | 20 | 2 | 2 | 4 | 9986 | 7988 | 5405 | 10123 | 6789 | >10 | ND | >10 |
| 11 | IGHV10-1 | IGHD1-1 | IGHJ1 | VRDYGYGYFDD | 5 | 7 | 5 | IGKV12-44 | IGKJ5 | QHHYGAPLT | 24 | 7 | 6 | 4 | 9142 | 10593 | 6055 | 9243 | 4475 | >10 | ND | >10 |
| 26 | IGHV10-1 | IGHD1-1 | IGHJ1 | VRDYGYGYFDV | 3 | 10 | 7 | IGKV12-44 | IGKJ5 | QHHYGTPLT | 20 | 2 | 2 | 4 | 11272 | 8277 | 5080 | 9841 | 5567 | >10 | ND | >10 |
| 6 | IGHV10-1 | IGHD1-1 | IGHJ1 | VRDYGYGYFDV | 3 | 3 | 3 | IGKV12-44 | IGKJ5 | QHHYGTPLT | 20 | 5 | 3 | 4 | 8831 | 6909 | 3904 | 7408 | 4130 | ND | ND | ND |
| 22 | IGHV10-1 | IGHD1-1 | IGHJ1 | VRDYGYGYFDV | 3 | 5 | 5 | IGKV12-44 | IGKJ5 | QHHYGTPLT | 20 | 7 | 4 | 4 | 13567 | 13833 | 6531 | 7301 | 8483 | ND | ND | ND |
| 260 | IGHV14-1 | IGHD1-1 | IGHJ3 | AIITTVLPWFAY | 6 | 5 | 4 | IGKV8-27 | IGKJ1 | HQYLSSWT | 25 | 3 | 1 | 3 | 3375 | 5234 | 2946 | 1222 | 918 | >10 | ND | >10 |
| 266 | IGHV14-1 | IGHD1-1 | IGHJ3 | AIMTTVLPWFAY | 7 | 4 | 2 | IGKV8-27 | IGKJ1 | HQYLSSWT | 25 | 8 | 2 | 3 | 7088 | 8459 | 6254 | 4139 | 1969 | >10 | ND | >10 |
| 267 | IGHV14-1 | IGHD1-1 | IGHJ3 | AIITTVLPWFAY | 6 | 5 | 1 | IGKV8-27 | IGKJ1 | HQYLSSWT | 25 | 6 | 3 | 3 | 6393 | 7028 | 5196 | 1569 | 2057 | >10 | ND | >10 |
| 268 | IGHV14-1 | IGHD1-1 | IGHJ3 | AIITTVAPFFAY | 8 | 2 | 1 | IGKV8-27 | IGKJ1 | HQYLSSWT | 25 | 10 | 6 | 3 | 5148 | 7437 | 5846 | 2716 | 1816 | >10 | ND | >10 |
| 269 | IGHV14-1 | IGHD1-1 | IGHJ3 | AIITTVAPYFAY | 9 | 3 | 2 | IGKV8-27 | IGKJ1 | HQYLSSWT | 25 | 1 | 1 | 3 | 4428 | 5801 | 4692 | 2511 | 1945 | >10 | >10 | >10 |
| 272 | IGHV14-1 | IGHD1-1 | IGHJ3 | ALITTVLPWFAF | 10 | 7 | 5 | IGKV8-27 | IGKJ1 | HQYLSSWT | 25 | 5 | 3 | 3 | 5578 | 6755 | 4382 | 2122 | 1487 | >10 | >10 | >10 |
| 262 | IGHV14-1 | IGHD1-1 | IGHJ3 | AIMTTVLPWFAY | 7 | 4 | 4 | IGKV8-27 | IGKJ1 | HQYLSSWT | 25 | 3 | 0 | 3 | 604 | 2368 | 1366 | 416 | 778 | >10 | >10 | >10 |
| 274 | IGHV14-1 | IGHD1-1 | IGHJ3 | ALITTVLPWFTF | 11 | 6 | 5 | IGKV8-27 | IGKJ1 | HQYLSSWT | 25 | 2 | 2 | 3 | 2258 | 5693 | 3213 | 1517 | 2488 | >10 | >10 | >10 |
| 4 | IGHV5-15 | IGHD2-4 | IGHJ2 | TRESDYGFFDY | 12 | 3 | 1 | IGKV12-44 | IGKJ1 | QHHYGSPPWT | 26 | 7 | 5 | 4 | 1696 | 1371 | 2286 | 1709 | 576 | >10 | >10 | >10 |
| 7 | IGHV5-15 | IGHD2-4 | IGHJ2 | ARESDYGFFDC | 13 | 2 | 1 | IGKV12-44 | IGKJ1 | QHHYGTPPWT | 27 | 6 | 4 | 4 | 4637 | 4151 | 5009 | 4802 | 1952 | >10 | >10 | >10 |
| 37 | IGHV5-15 | IGHD2-4 | IGHJ2 | VRESDYGYFAY | 14 | 1 | 1 | IGKV12-44 | IGKJ1 | QHHYGTPPWT | 27 | 5 | 4 | 4 | 4961 | 5751 | 6834 | 5525 | 2144 | >10 | >10 | >10 |
| 35 | IGHV5-15 | IGHD2-4 | IGHJ2 | ARESDYGFFDY | 15 | 2 | 2 | IGKV12-44 | IGKJ1 | QHHYGSPPWT | 26 | 5 | 5 | 4 | 3599 | 2630 | 5035 | 4983 | 1154 | >10 | ND | >10 |
| 15 | IGHV2-6-1 | IGHD1-1 | IGHJ4 | ARHGGTYYYGISPYAMDY | 16 | 5 | 3 | IGKV12-46 | IGKJ2 | QHFWDTPYT | 28 | 3 | 2 | 4 | 16395 | 25223 | 24162 | 13251 | 10761 | 0.283 | 0.334 | 0.885 |
| 25 | IGHV2-6-1 | IGHD1-1 | IGHJ4 | ARHSGTYYYGSSPYAMDY | 17 | 11 | 8 | IGKV12-46 | IGKJ2 | QHFWDTPYT | 28 | 3 | 3 | 4 | 15040 | 24927 | 25244 | 13179 | 10933 | 0.135 | 0.338 | 0.703 |
| 47 | IGHV2-6-1 | IGHD1-1 | IGHJ4 | ARHSGTYYYGSSPYGMDY | 18 | 8 | 3 | IGKV12-46 | IGKJ2 | QHFWDTPYT | 28 | 4 | 3 | 4 | 9698 | 10084 | 13382 | 5897 | 3451 | 0.267 | 2.83 | 4.36 |
| 1 | IGHV2-9 | IGHD2-2 | IGHJ2 | ARGWLGHYFDY | 19 | 9 | 5 | IGKV6-17 | IGKJ1 | QQHYSTPRT | 29 | 2 | 2 | 4 | 20838 | 23305 | 21280 | 27407 | 6318 | >10 | ND | >10 |

Fig. 12-2

| mAb ID | HC | | | | | | | LC | | | | | | Class | Bead-based flow cytometry assay | | | | | IC50 (microg/ml) | | |
|---|---|---|---|---|---|---|---|---|---|---|---|---|---|---|---|---|---|---|---|---|---|---|
| | IGHV | IGHD | IGHJ | CDR3 | SEQ ID NO. | SHM | AA mut# | IGKV | IGKJ | CDR3 | SEQ ID NO. | SHM | AA mut# | | CoV1 gMFI | WIV1 gMFI | CoV2 gMFI | AL103 (clade2) gMFI | BM48-31 (clade3) gMFI | CoV1 | WIV1 | CoV2 |
| GM9-2 | | | | | | | | | | | | | | | | | | | | | | |
| 50 | IGHV1S16 | IGHD3-3 | IGHJ2 | TIGYGYGYFDY | 30 | 11 | 8 | IGKV12-44 | IGKJ2 | QHHYGTPYT | 41 | 5 | 3 | 4 | 16122 | 17688 | 12497 | 21104 | 6181 | >10 | >10 | >10 |
| 53 | IGHV1S16 | IGHD3-3 | IGHJ2 | TIGYGYGYFDY | 30 | 14 | 7 | IGKV12-44 | IGKJ2 | QHHYGTPYT | 41 | 5 | 3 | 4 | 12771 | 17556 | 11699 | 18156 | 6010 | >10 | >10 | >10 |
| 61 | IGHV1S16 | IGHD3-3 | IGHJ2 | TIGYGYGYFDY | 30 | 15 | 10 | IGKV12-44 | IGKJ2 | QHHYGTPYT | 41 | 8 | 7 | 4 | 14321 | 11556 | 9210 | 13600 | 5239 | >10 | ND | >10 |
| 86 | IGHV1S16 | IGHD3-3 | IGHJ2 | TKGYGYGYFDY | 31 | 17 | 12 | IGKV12-44 | IGKJ2 | QHHYGSPYT | 42 | 7 | 5 | 4 | 15673 | 8716 | 8832 | 12444 | 3488 | >10 | ND | >10 |
| 96 | IGHV1S16 | IGHD3-3 | IGHJ2 | TKGYGYGYFDY | 31 | 18 | 12 | IGKV12-44 | IGKJ2 | QHHYGSPYT | 42 | 10 | 5 | 4 | 11943 | 8079 | 6560 | 7040 | 1899 | >10 | ND | >10 |
| 68 | IGHV1S16 | IGHD3-3 | IGHJ2 | TKGYGYGYFDQ | 32 | 17 | 12 | IGKV12-44 | IGKJ2 | QHHYGSPYT | 42 | 5 | 3 | 4 | 12144 | 7547 | 7338 | 10375 | 2421 | >10 | >10 | >10 |
| 58 | IGHV1S16 | IGHD3-3 | IGHJ2 | TKGYGYGYFDY | 31 | 21 | 16 | IGKV12-44 | IGKJ2 | QHHYGSPYT | 42 | 11 | 6 | 4 | 14868 | 15838 | 10993 | 18306 | 5667 | >10 | >10 | >10 |
| 65 | IGHV1S16 | IGHD3-3 | IGHJ2 | TIGYGYGYFDY | 30 | 13 | 8 | IGKV12-44 | IGKJ2 | QHHYGTPYT | 41 | 4 | 3 | 4 | 18241 | 18934 | 14010 | 24078 | 11685 | >10 | ND | >10 |
| 78 | IGHV1S16 | IGHD3-3 | IGHJ2 | SIGYGYGYFDY | 33 | 12 | 7 | IGKV12-44 | IGKJ2 | QHHYGTPYT | 41 | 4 | 3 | 4 | 19050 | 20688 | 13192 | 23283 | 7559 | >10 | >10 | >10 |
| 88 | IGHV1S16 | IGHD3-3 | IGHJ2 | TIGYGYGYFDY | 30 | 14 | 11 | IGKV12-44 | IGKJ2 | QHHYGTPYT | 41 | 11 | 9 | 4 | 14076 | 17832 | 11546 | 16873 | 5904 | >10 | >10 | >10 |
| 59 | IGHV1S16 | IGHD3-3 | IGHJ2 | TKGYGYGYFDY | 31 | 21 | 16 | IGKV12-44 | IGKJ2 | QHHYGSPYT | 42 | 11 | 6 | 4 | 14194 | 9701 | 10172 | 13054 | 4551 | >10 | ND | >10 |
| 76 | IGHV1S16 | IGHD3-3 | IGHJ2 | TIGYGYGYFDY | 30 | 13 | 8 | IGKV12-44 | IGKJ2 | QHHYGTPYT | 41 | 10 | 8 | 4 | 14541 | 12622 | 10912 | 14996 | 6408 | >10 | ND | >10 |
| 80 | IGHV1S16 | IGHD3-3 | IGHJ1 | TIGYGYGYFDY | 30 | 17 | 10 | IGKV12-44 | IGKJ2 | QHHYGSPYT | 42 | 5 | 3 | 4 | 7560 | 3933 | 5790 | 5724 | 453 | >10 | ND | >10 |
| 93 | IGHV1S16 | IGHD3-3 | IGHJ2 | TIGYGYGYFDY | 30 | 11 | 8 | IGKV12-44 | IGKJ2 | QHHYGTPYT | 41 | 10 | 8 | 4 | 12998 | 9003 | 7611 | 11942 | 3576 | >10 | ND | >10 |
| 98 | IGHV1S16 | IGHD3-3 | IGHJ2 | TIGYGYGYFDY | 30 | 11 | 7 | IGKV12-44 | IGKJ2 | QHHYGTPYT | 41 | 7 | 3 | 4 | 13925 | 10162 | 10672 | 14466 | 3945 | >10 | ND | >10 |
| 54 | IGHV1-80 | - | IGHJ1 | ARNFDV | 34 | 16 | 7 | IGKV4-68 | IGKJ2 | QQWNNYPYT | 43 | 7 | 5 | 4 | 12247 | 15272 | 19638 | 4109 | 57.9 | >10 | >10 | >10 |
| 57 | IGHV1-80 | - | IGHJ1 | ARYFDV | 35 | 16 | 7 | IGKV4-68 | IGKJ2 | QQWSSYPYT | 44 | 2 | 1 | 4 | 5256 | 5082 | 7172 | 837 | 61.6 | >10 | ND | >10 |
| 64 | IGHV1-80 | - | IGHJ1 | ARYFDV | 35 | 19 | 10 | IGKV4-68 | IGKJ2 | QQWNNYPYT | 43 | 2 | 1 | 4 | 19103 | 17498 | 20084 | 9511 | 70.2 | >10 | ND | >10 |
| 72 | IGHV1-80 | - | IGHJ1 | ARYFDV | 35 | 20 | 8 | IGKV4-68 | IGKJ2 | QQWNNYPYT | 43 | 5 | 4 | 4 | 17263 | 19896 | 21288 | 8625 | 119 | >10 | ND | >10 |
| 91 | IGHV1-80 | - | IGHJ1 | ARYFDV | 35 | 18 | 8 | IGKV4-68 | IGKJ2 | QQWSSYPYT | 44 | 3 | 1 | 4 | 14998 | 16431 | 18234 | 7858 | 95.8 | >10 | ND | >10 |
| 77 | IGHV1-80 | - | IGHJ1 | ARYFDV | 35 | 18 | 7 | IGKV4-68 | IGKJ2 | QQWNNYPYT | 43 | 6 | 2 | 4 | 19871 | 22576 | 22090 | 9744 | 316 | ND | ND | ND |
| 101 | IGHV1-80 | - | IGHJ1 | ARYFDV | 35 | 15 | 7 | IGKV4-68 | IGKJ2 | QQWNNYPYT | 43 | 4 | 2 | 4 | 16593 | 17578 | 19958 | 6894 | 78.1 | >10 | ND | >10 |
| 79 | IGHV1-82 | IGHD1-1 | IGHJ1 | VRDYGYGYFDV | 3 | 21 | 13 | IGKV12-44 | IGKJ2 | QHHYGPPYT | 45 | 12 | 7 | 4 | 10826 | 9254 | 8657 | 13704 | 3653 | >10 | ND | >10 |
| 95 | IGHV1-82 | IGHD1-1 | IGHJ1 | VRDYGYGYFDV | 3 | 18 | 10 | IGKV12-44 | IGKJ2 | QHHYGPPFT | 46 | 11 | 8 | 4 | 5772 | 3860 | 4641 | 6229 | 1234 | >10 | >10 | >10 |
| 51 | IGHV1-82 | IGHD1-1 | IGHJ1 | VRDYGYGYFGV | 36 | 14 | 7 | IGKV12-44 | IGKJ2 | QHHYGPPYT | 45 | 8 | 6 | 4 | 8578 | 2120 | 3932 | 5386 | 533 | >10 | ND | >10 |
| 67 | IGHV1-82 | IGHD1-1 | IGHJ1 | VRDYGYGYFDD | 5 | 17 | 10 | IGKV12-44 | IGKJ2 | QHHYGPPYT | 45 | 10 | 8 | 4 | 10166 | 7017 | 6614 | 10057 | 3302 | >10 | ND | >10 |
| 71 | IGHV1-82 | IGHD1-1 | IGHJ1 | VRDYGYGYFDV | 3 | 14 | 7 | IGKV12-44 | IGKJ2 | QHHYGPPYT | 45 | 10 | 8 | 4 | 14059 | 9333 | 9480 | 12308 | 3932 | >10 | ND | >10 |
| 84 | IGHV1-82 | IGHD1-1 | IGHJ1 | VRDYGYGYFDV | 3 | 11 | 6 | IGKV12-44 | IGKJ2 | QHHYGPPYT | 45 | 11 | 8 | 4 | 12342 | 6950 | 8095 | 11533 | 2622 | >10 | ND | >10 |
| 100 | IGHV1-82 | IGHD1-1 | IGHJ1 | VRDYGYGYFDV | 3 | 15 | 7 | IGKV12-44 | IGKJ2 | QHHYGPPYT | 45 | 9 | 6 | 4 | 10502 | 6290 | 7364 | 9944 | 3017 | >10 | ND | >10 |
| 87 | IGHV1S16 | IGHD1-2 | IGHJ1 | TIGFGYGYFDY | 37 | 13 | 9 | IGKV1-117 | IGKJ1 | FQGSHVPRX | 47 | 0 | 0 | ND | 21.4 | 28 | 24.4 | 58.2 | 74.9 | >10 | ND | >10 |
| 276 | IGHV5-12-2 | IGHD2-4 | IGHJ4 | ARHYDYDDGMDY | 38 | 5 | 5 | IGKV3-4 | IGKJ1 | QQGGEDPWT | 48 | 6 | 5 | ND | 82.7 | 47.8 | 49.2 | 50.7 | 51.7 | ND | ND | ND |
| 277 | IGHV14-1 | IGHD2-1 | IGHJ1 | ARYGNDWYFDV | 39 | 4 | 3 | IGKV3-4 | IGKJ1 | QQGGEDPWT | 48 | 3 | 3 | ND | 63.1 | 61.8 | 52.8 | 34.8 | 62.3 | ND | ND | ND |
| 280 | IGHV14-3 | IGHD2-3 | IGHJ4 | TTPVYDGYYDYAMDC | 40 | 7 | 4 | IGKV12-44 | IGKJ2 | QHHYGPPYT | 45 | 13 | 9 | ND | 52.9 | 74 | 208 | 53.8 | 75.1 | >10 | ND | >10 |

Fig. 12-3

| mAb ID | HC | | | | | | | LC | | | | | | Class | Bead-based flow cytomery assay | | | | | IC50 (microg/ml) | | |
|---|---|---|---|---|---|---|---|---|---|---|---|---|---|---|---|---|---|---|---|---|---|---|
| | IGHV | IGHD | IGHJ | CDR3 | SEQ ID NO. | SHM | AA mut# | IGKV | IGKJ | CDR3 | SEQ ID NO. | SHM | AA mut# | | CoV1 gMFI | WIV1 gMFI | CoV2 gMFI | AL103 (clade2) gMFI | BM48-31 (clade3) gMFI | CoV1 | WIV1 | CoV2 |
| GM14-1 | | | | | | | | | | | | | | | | | | | | | | |
| 105 | IGHV14-3 | IGHD3-2 | IGHJ4 | ARLDSSYYVDYPMDY | 49 | 11 | 5 | IGKV14-111 | IGKJ2 | LQYDEFPYT | 71 | 6 | 3 | 4 | 5409 | 6880 | 11691 | 72.2 | 67.4 | 2.94 | 8.43 | >10 |
| 108 | IGHV14-3 | IGHD3-2 | IGHJ4 | ARLDSSYYVDYPMDY | 49 | 8 | 6 | IGKV14-111 | IGKJ2 | LQYDEFPYT | 71 | 8 | 6 | 4 | 7852 | 9929 | 10110 | 72.6 | 76.5 | 6.10 | 5.99 | >10 |
| 110 | IGHV14-3 | IGHD3-2 | IGHJ4 | ARLDSSYYVDYPLDY | 50 | 8 | 6 | IGKV14-111 | IGKJ2 | LQYDEFPYT | 71 | 4 | 2 | 4 | 1349 | 2019 | 2597 | 44.4 | 60.8 | >10 | ND | >10 |
| 112 | IGHV14-3 | IGHD3-2 | IGHJ4 | ARLDSSAYVDYPMDY | 51 | 8 | 6 | IGKV14-111 | IGKJ2 | LQYDEFPFT | 72 | 3 | 2 | 4 | 8268 | 12660 | 14247 | 81.9 | 76.1 | >10 | >10 | >10 |
| 113 | IGHV14-3 | IGHD3-2 | IGHJ4 | ARLDSSAYVDYPMDY | 51 | 5 | 4 | IGKV14-111 | IGKJ2 | LQYDEFPFT | 72 | 6 | 3 | 4 | 9974 | 11348 | 11745 | 67.7 | 73.4 | 1.50 | 3.09 | >10 |
| 115 | IGHV14-3 | IGHD3-2 | IGHJ4 | VRLDSSAYVGYPMDY | 52 | 7 | 2 | IGKV14-111 | IGKJ2 | LQYDEFPYT | 71 | 5 | 4 | 4 | 9224 | 13606 | 10805 | 74.3 | 82.5 | 10.0 | 9.14 | >10 |
| 116 | IGHV14-3 | IGHD3-2 | IGHJ4 | ARLDSSYYVDYPMDY | 49 | 8 | 4 | IGKV14-111 | IGKJ2 | LQYDEFPYT | 71 | 5 | 3 | 4 | 6387 | 9601 | 6934 | 73.1 | 79.1 | ND | ND | ND |
| 117 | IGHV14-3 | IGHD3-2 | IGHJ4 | ARLDTSSYVDYAMDY | 53 | 7 | 5 | IGKV14-111 | IGKJ2 | LQYDEFPFT | 72 | 4 | 3 | 4 | 5545 | 8531 | 9670 | 77.1 | 78.1 | ND | ND | ND |
| 119 | IGHV14-3 | IGHD3-2 | IGHJ4 | ARLDSSYYVDYPMDY | 49 | 10 | 7 | IGKV14-111 | IGKJ2 | LQYDEFPYT | 71 | 4 | 1 | 4 | 2324 | 6438 | 6360 | 61.3 | 50.1 | 2.47 | 3.29 | >10 |
| 120 | IGHV14-3 | IGHD3-2 | IGHJ4 | ARLDSSYYVDYPMDY | 49 | 8 | 4 | IGKV14-111 | IGKJ2 | LQYDEFPYT | 71 | 5 | 2 | 4 | 4085 | 7506 | 6561 | 74.4 | 86.5 | ND | ND | >10 |
| 122 | IGHV14-3 | IGHD3-2 | IGHJ4 | ARLDSSAYVEYPMDY | 54 | 3 | 1 | IGKV14-111 | IGKJ2 | LQYDEFPFT | 72 | 7 | 5 | 4 | 9559 | 13144 | 13240 | 2561 | 104 | 4.87 | 0.437 | >10 |
| 125 | IGHV14-3 | IGHD3-2 | IGHJ4 | ARLDSSYYVDYAMDY | 55 | 10 | 10 | IGKV14-111 | IGKJ2 | LQYDEFPYT | 71 | 5 | 4 | 4 | 2601 | 4200 | 6791 | 66 | 83.2 | >10 | ND | >10 |
| 126 | IGHV14-3 | IGHD3-2 | IGHJ4 | ARLDSSAYVGYAMDY | 56 | 6 | 4 | IGKV14-111 | IGKJ2 | LQYDEFPYT | 71 | 9 | 7 | 4 | 2470 | 3100 | 5240 | 64.7 | 56.2 | >10 | ND | >10 |
| 129 | IGHV14-3 | IGHD3-2 | IGHJ4 | ARLDSSAYVDYPMDF | 57 | 6 | 4 | IGKV14-111 | IGKJ2 | LQYDEFPYT | 71 | 4 | 4 | 4 | 8998 | 13167 | 11271 | 67.3 | 64.2 | 4.87 | 1.27 | 5.00 |
| 130 | IGHV14-3 | IGHD3-2 | IGHJ4 | ARLDSSAYLGYPLDY | 58 | 8 | 8 | IGKV14-111 | IGKJ2 | LQYDEFPYT | 71 | 7 | 6 | 4 | 9274 | 11361 | 12316 | 68.9 | 71 | 2.09 | 4.06 | 4.93 |
| 131 | IGHV14-3 | IGHD3-2 | IGHJ4 | ARLDSSYYVDYPMDY | 49 | 11 | 8 | IGKV14-111 | IGKJ2 | LQYDEFPYT | 71 | 3 | 2 | 4 | 6031 | 8565 | 8808 | 69.5 | 75.1 | >10 | 2.82 | >10 |
| 150 | IGHV14-3 | IGHD3-2 | IGHJ4 | ARLDSSGYVGYAMDY | 59 | 6 | 6 | IGKV14-111 | IGKJ2 | QQYDEFPYT | 73 | 9 | 5 | 4 | 751 | 2882 | 4109 | 51.4 | 51.6 | >10 | >10 | >10 |
| 151 | IGHV14-3 | IGHD3-2 | IGHJ4 | ARLDSAAYVGYAMDY | 60 | 17 | 10 | IGKV14-111 | IGKJ2 | LQYDEFPYT | 71 | 11 | 6 | 4 | 482 | 1646 | 1382 | 60.8 | 80 | ND | ND | ND |
| 154 | IGHV14-3 | IGHD3-2 | IGHJ4 | IRLDSSYYVDYPVDY | 61 | 11 | 8 | IGKV14-111 | IGKJ2 | LQYDEFPYT | 71 | 5 | 4 | 4 | 4859 | 10130 | 8566 | 89.1 | | ND | ND | ND |
| 155 | IGHV14-3 | IGHD3-2 | IGHJ4 | ARLDSSYYVDYPVDY | 62 | 9 | 7 | IGKV14-111 | IGKJ2 | LQYDEFPYT | 71 | 5 | 4 | 4 | 5933 | 11292 | 8928 | 68.4 | 66.7 | 1.06 | 9.74 | >10 |
| 160 | IGHV14-3 | IGHD3-2 | IGHJ4 | ARLDISDYVDYPMDH | 63 | 5 | 4 | IGKV14-111 | IGKJ2 | LQYDEFPYT | 71 | 6 | 6 | 4 | 5406 | 10441 | 9372 | 655 | 910 | 2.29 | 3.43 | >10 |
| 161 | IGHV14-3 | IGHD3-2 | IGHJ4 | ARLDSSYYVDYPMDY | 49 | 8 | 6 | IGKV14-111 | IGKJ2 | LQYDEFPYT | 71 | 5 | 4 | 4 | 5690 | 7853 | 9292 | 98.7 | 82.7 | >10 | 5.75 | >10 |
| 164 | IGHV14-3 | IGHD3-2 | IGHJ4 | ARLDSSYYVDYPMDY | 49 | 8 | 5 | IGKV14-111 | IGKJ2 | LQYDEFPYT | 71 | 4 | 2 | 4 | 4638 | 7786 | 9208 | 71.3 | 83.5 | 4.97 | >10 | >10 |
| 281 | IGHV14-3 | IGHD3-2 | IGHJ4 | VRLDSSYYVDYPMDY | 52 | 12 | 7 | IGKV14-111 | IGKJ2 | LQYDEFPYT | 71 | 10 | 9 | 4 | 14507 | 16084 | 12614 | 355 | 68.2 | >10 | >10 | >10 |
| 282 | IGHV14-3 | IGHD3-2 | IGHJ4 | ARLDSSYVDYPMDY | 64 | 8 | 5 | IGKV14-111 | IGKJ2 | LQYDEFPYT | 71 | 9 | 7 | 4 | 17854 | 19935 | 12653 | 81.5 | 77.9 | 10.0 | 1.50 | >10 |
| 283 | IGHV14-3 | IGHD3-2 | IGHJ4 | ARLDSSAYVGYPMDY | 65 | 10 | 5 | IGKV14-111 | IGKJ2 | LQYDDFPYT | 74 | 7 | 7 | 4 | 11088 | 14890 | 12553 | 516 | 64 | 1.88 | 1.10 | >10 |
| 284 | IGHV14-3 | IGHD3-2 | IGHJ4 | ARLDSSYYVDYPMDY | 49 | 8 | 7 | IGKV14-111 | IGKJ2 | LQYAEFPYT | 75 | 7 | 5 | 4 | 11932 | 13852 | 16684 | 65.5 | 69.8 | ND | ND | ND |
| 285 | IGHV14-3 | IGHD3-2 | IGHJ4 | ARLDSSYYVDYPMDY | 49 | 7 | 5 | IGKV14-111 | IGKJ2 | LHYDEFPYT | 76 | 5 | 3 | 4 | 7547 | 8736 | 11231 | 54.8 | 66.3 | >10 | >10 | ND |
| 286 | IGHV14-3 | IGHD3-2 | IGHJ4 | ARLDSSYYVDYPLDY | 50 | 7 | 6 | IGKV14-111 | IGKJ2 | LQYDEFPYT | 71 | 4 | 2 | 4 | 1503 | 2302 | 3355 | 45.1 | 51.5 | ND | ND | ND |
| 289 | IGHV14-3 | IGHD3-2 | IGHJ4 | ARLDSSAYVGYAMDY | 56 | 13 | 7 | IGKV14-111 | IGKJ2 | LQYDEFPYT | 71 | 6 | 4 | 4 | 1175 | 2505 | 2579 | 65.2 | 76.4 | >10 | 2.39 | ND |
| 290 | IGHV14-3 | IGHD3-2 | IGHJ4 | ARLDSSCYVDYPVDY | 66 | 11 | 8 | IGKV14-111 | IGKJ2 | LQYDEFPYT | 71 | 5 | 4 | 4 | 12357 | 20172 | 16169 | 69.9 | 81.4 | ND | ND | ND |
| 291 | IGHV14-3 | IGHD3-2 | IGHJ4 | ARLDSSYYVDYPMDY | 49 | 8 | 6 | IGKV14-111 | IGKJ2 | LQYDEFPYT | 71 | 6 | 4 | 4 | 9010 | 11177 | 11143 | 64.6 | 66.1 | >10 | >10 | >10 |
| 292 | IGHV14-3 | IGHD3-2 | IGHJ4 | ARLDSSYYVDYPMDY | 49 | 10 | 8 | IGKV14-111 | IGKJ2 | LQYDEFPYT | 71 | 5 | 4 | 4 | 346 | 477 | 2301 | 69.8 | 95.2 | >10 | >10 | >10 |
| 159 | IGHV14-3 | IGHD3-2 | IGHJ4 | ARLDSSAYVDYPMDF | 57 | 10 | 7 | IGKV14-111 | IGKJ2 | LQYDEFPYT | 71 | 4 | 4 | 4 | 7483 | 9054 | 6715 | 86.6 | 79.1 | ND | ND | ND |
| 121-2 | IGHV14-3 | IGHD3-2 | IGHJ4 | ARLDSSYYVDYPMDY | 49 | 10 | 6 | IGKV14-111 | IGKJ2 | LQYDEFPYT | 71 | 5 | 3 | 4 | 9723 | 9687 | 9327 | 44.7 | 23.2 | ND | ND | ND |
| 156-2 | IGHV14-3 | IGHD3-2 | IGHJ4 | ARLDSSYFVDYPLDS | 67 | 6 | 5 | IGKV14-111 | IGKJ2 | LQYDEFPYT | 71 | 4 | 2 | 4 | 5217 | 6551 | 7877 | 38.6 | 31.9 | ND | ND | ND |
| 158-4 | IGHV14-3 | IGHD3-2 | IGHJ4 | ARLDSSYYVDYPMDY | 49 | 11 | 8 | IGKV14-111 | IGKJ2 | LQYDEFPYT | 71 | 5 | 4 | 4 | 7250 | 6816 | 8577 | 37.9 | 35.4 | ND | ND | ND |
| 114-2 | IGHV14-3 | IGHD3-2 | IGHJ4 | VRLDSSYYVDYPVDY | 68 | 12 | 8 | IGKV14-111 | IGKJ2 | LQYDEFPYT | 71 | 5 | 4 | 4 | 26.2 | 331 | 211 | 38 | 45.9 | ND | ND | ND |
| 107 | IGHV5-6 | IGHD2-14 | IGHJ4 | ARQRGSYYRYDDYAMDY | 69 | 6 | 5 | IGKV1-117 | IGKJ1 | FQGSHVPWT | 77 | 1 | 0 | 4 | 7126 | 8938 | 8013 | 10550 | 6234 | ND | ND | ND |
| 106 | IGHV5-6 | IGHD2-14 | IGHJ4 | ARQRGSYYRYDDYAMDY | 69 | 5 | 5 | IGKV1-117 | IGKJ1 | FQGSHVPWT | 77 | 2 | 1 | 4 | 7760 | 10150 | 9396 | 8711 | 5525 | >10 | ND | >10 |
| 165 | IGHV1-7 | IGHD2-1 | IGHJ4 | ARWGNYVSYYTMDY | 70 | 12 | 10 | IGKV4-61 | IGKJ1 | QHYHSYPPMWT | 78 | 4 | 3 | 4 | 20602 | 27407 | 18612 | 21860 | 20844 | >10 | ND | >10 |
| 157 | IGHV1-80 | - | IGHJ1 | ARYFDV | 35 | 15 | 5 | IGKV4-68 | IGKJ2 | QQWSSYPYT | 44 | 3 | 0 | 4 | 12197 | 17182 | 15593 | 9236 | 79.9 | >10 | >10 | >10 |

Fig. 12-4

| mAb ID | IGHV | IGHD | IGHJ | HC CDR3 | HC SEQ ID NO. | HC SHM | HC AA mut# | IGKV | IGKJ | LC CDR3 | LC SEQ ID NO. | LC SHM | LC AA mut# | Class | CoV1 gMFI | WIV1 gMFI | CoV2 gMFI | AL103 (clade2) gMFI | BM48-31 (clade3) gMFI | IC50 CoV1 | IC50 WIV1 | IC50 CoV2 |
|---|---|---|---|---|---|---|---|---|---|---|---|---|---|---|---|---|---|---|---|---|---|---|
| GM14-2 | | | | | | | | | | | | | | | | | | | | | | |
| 132 | IGHV14-3 | IGHD2-3 | IGHJ4 | ARLDYDGYYEYAMDY | 79 | 4 | 2 | IGKV14-111 | IGKJ1 | LQYDEFPRT | 104 | 2 | 1 | 4 | 4464 | 6994 | 8765 | 61.9 | 82.6 | 0.942 | 2.96 | 7.63 |
| 134 | IGHV14-3 | IGHD2-3 | IGHJ4 | ARLDYDTYYEYAMDY | 80 | 6 | 5 | IGKV14-111 | IGKJ1 | LQYDEFPRT | 104 | 3 | 2 | 4 | 3874 | 4863 | 9427 | 67.5 | 48.4 | >10 | >10 | >10 |
| 135 | IGHV14-3 | IGHD2-3 | IGHJ4 | ARLDFDGYYEYPMDY | 81 | 3 | 3 | IGKV14-111 | IGKJ1 | LQYDEFPRT | 104 | 4 | 4 | 4 | 4933 | 7343 | 9713 | 444 | 519 | 0.403 | 4.31 | >10 |
| 136 | IGHV14-3 | IGHD2-3 | IGHJ4 | SRLDFDGYYEYAMDY | 82 | 11 | 8 | IGKV14-111 | IGKJ1 | LQYDEFPRT | 104 | 1 | 1 | 4 | 2442 | 3289 | 5488 | 63.6 | 69.6 | 3.00 | 9.26 | >10 |
| 137 | IGHV14-3 | IGHD2-3 | IGHJ4 | TRLDYDGYYDYAVDY | 83 | 8 | 8 | IGKV14-111 | IGKJ1 | LQYDEFPRT | 104 | 1 | 0 | 4 | 3839 | 4172 | 12486 | 81.1 | 61.5 | 4.96 | 1.49 | >10 |
| 141 | IGHV14-3 | IGHD2-3 | IGHJ4 | TRLDYDGYYDYAMDY | 84 | 4 | 4 | IGKV14-111 | IGKJ1 | LQYDEFPRT | 104 | 4 | 4 | 4 | 2421 | 3250 | 5003 | 47.5 | 62.6 | ND | ND | ND |
| 144 | IGHV14-3 | IGHD2-3 | IGHJ4 | ARLDYDGYYDFAMDY | 85 | 6 | 6 | IGKV14-111 | IGKJ1 | LQYDEFPRT | 104 | 8 | 4 | 4 | 2025 | 2973 | 6286 | 65.3 | 61.4 | ND | ND | ND |
| 148 | IGHV14-3 | IGHD2-3 | IGHJ4 | ARLDYDGYYEYPMDY | 86 | 6 | 6 | IGKV14-111 | IGKJ1 | LQYDEFPRT | 104 | 4 | 4 | 4 | 266 | 256 | 1025 | 66.7 | 50.8 | ND | ND | ND |
| 168 | IGHV14-3 | IGHD2-3 | IGHJ4 | ARLDYDGYYENAMDY | 87 | 4 | 3 | IGKV14-111 | IGKJ1 | LQYDEFPRT | 104 | 8 | 4 | 4 | 2389 | 5670 | 9601 | 72.6 | 94.6 | ND | ND | ND |
| 169 | IGHV14-3 | IGHD2-3 | IGHJ4 | ARLDYAYYEYAMDY | 88 | 5 | 5 | IGKV14-111 | IGKJ1 | LQYDEFPRT | 104 | 2 | 1 | 4 | 5043 | 8943 | 11464 | 62.7 | 417 | 8.79 | 2.80 | >10 |
| 170 | IGHV14-3 | IGHD2-3 | IGHJ4 | ARLDYAGYYEYAMDY | 89 | 4 | 4 | IGKV14-111 | IGKJ1 | LQYDEFPRT | 104 | 5 | 3 | 4 | 610 | 1341 | 1585 | 71.2 | 70.5 | ND | ND | ND |
| 171 | IGHV14-3 | IGHD2-3 | IGHJ4 | TRLDYEGYYEYAMDY | 90 | 7 | 5 | IGKV14-111 | IGKJ1 | LQYDEFPRT | 104 | 2 | 2 | 4 | 3889 | 4214 | 5354 | 264 | 69.9 | 2.92 | 9.68 | >10 |
| 172 | IGHV14-3 | IGHD2-3 | IGHJ4 | TRLDYDSYYEYALDY | 91 | 13 | 7 | IGKV14-111 | IGKJ1 | LQYDEFPRT | 104 | 6 | 3 | 4 | 3114 | 4426 | 3440 | 51.6 | 87.5 | ND | ND | ND |
| 173 | IGHV14-3 | IGHD2-3 | IGHJ4 | ARLDYDGYYEYAMDY | 92 | 3 | 3 | IGKV14-111 | IGKJ1 | LQYDEFPRT | 104 | 5 | 3 | 4 | 2114 | 3614 | 4144 | 73.6 | 67.6 | ND | ND | ND |
| 174 | IGHV14-3 | IGHD2-3 | IGHJ4 | ARLDYDAYYEYAMDY | 88 | 4 | 3 | IGKV14-111 | IGKJ1 | LQYDEFPRT | 104 | 1 | 1 | 4 | 283 | 519 | 816 | 66.3 | 52.8 | ND | ND | ND |
| 175 | IGHV14-3 | IGHD2-3 | IGHJ4 | SRLDYDGYYEYTMDY | 93 | 10 | 5 | IGKV14-111 | IGKJ1 | LQYDEFPRT | 104 | 3 | 3 | 4 | 1080 | 2058 | 2758 | 63.9 | 73.1 | ND | ND | ND |
| 178 | IGHV14-3 | IGHD2-3 | IGHJ4 | ARLDYDGYYDYALDY | 94 | 7 | 6 | IGKV14-111 | IGKJ1 | LQYDEFPRT | 104 | 6 | 3 | 4 | 806 | 1450 | 1323 | 59.1 | 79.2 | ND | ND | ND |
| 180 | IGHV14-3 | IGHD2-3 | IGHJ4 | ARLDYDGYYEYAMDY | 79 | 6 | 2 | IGKV14-111 | IGKJ1 | LQYDEFPRT | 104 | 1 | 1 | 4 | 3159 | 5010 | 5613 | 41.5 | 68.5 | ND | ND | ND |
| 182 | IGHV14-3 | IGHD2-3 | IGHJ4 | ARLDYDGYYEYAMDY | 79 | 2 | 1 | IGKV14-111 | IGKJ1 | LQYDEFPRT | 104 | 4 | 2 | 4 | 2496 | 4095 | 4583 | 46.4 | 82.7 | >10 | 3.23 | >10 |
| 183 | IGHV14-3 | IGHD2-3 | IGHJ4 | ARLDYDGYYEYPLDY | 95 | 9 | 7 | IGKV14-111 | IGKJ1 | LQYDEFPRT | 104 | 5 | 3 | 4 | 2526 | 3067 | 6266 | 61 | 69.8 | >10 | >10 | >10 |
| 185 | IGHV14-3 | IGHD2-3 | IGHJ4 | ARLDYDGYYDYAMDY | 96 | 5 | 5 | IGKV14-111 | IGKJ1 | LQYDEFPRT | 104 | 5 | 3 | 4 | 3918 | 7730 | 10141 | 86.3 | 829 | 2.31 | 5.99 | >10 |
| 186 | IGHV14-3 | IGHD2-3 | IGHJ4 | ARLDYDGYYEYAVDY | 97 | 4 | 4 | IGKV14-111 | IGKJ1 | LQYDEFPRT | 104 | 1 | 1 | 4 | 2188 | 4359 | 6143 | 71 | 79 | ND | ND | ND |
| 187 | IGHV14-3 | IGHD2-3 | IGHJ4 | ARLDYDGYYEYAMDY | 79 | 6 | 4 | IGKV14-111 | IGKJ1 | LQYDEFPRT | 104 | 6 | 3 | 4 | 1792 | 3397 | 6621 | 91.6 | 69.5 | >10 | 8.16 | >10 |
| 188 | IGHV14-3 | IGHD2-3 | IGHJ4 | ARLDYDGYYEYALDY | 98 | 5 | 4 | IGKV14-111 | IGKJ1 | LQYDEFPRT | 104 | 9 | 6 | 4 | 2383 | 3263 | 6266 | 69.6 | 68.1 | ND | ND | ND |
| 191 | IGHV14-3 | IGHD2-3 | IGHJ4 | ARLDYDGYYDFAMDY | 85 | 6 | 6 | IGKV14-111 | IGKJ1 | LQYDEFPRT | 104 | 4 | 3 | 4 | 1276 | 2492 | 2695 | 50 | 64.8 | ND | ND | ND |
| 192 | IGHV14-3 | IGHD2-3 | IGHJ4 | ARLDAYYENAMDY | 99 | 3 | 1 | IGKV14-111 | IGKJ1 | LQYDEFPRT | 104 | 1 | 1 | 4 | 2326 | 3652 | 6478 | 64.7 | 505 | ND | ND | ND |
| 193 | IGHV14-3 | IGHD1-2 | IGHJ2 | GRLDSLHYDYGFDY | 100 | 5 | 3 | IGKV14-111 | IGKJ1 | LQYDEFPYT | 71 | 5 | 5 | 4 | 3285 | 3406 | 6130 | 69.3 | 80.9 | >10 | >10 | >10 |
| 194 | IGHV14-3 | IGHD2-3 | IGHJ4 | VRLDVDGYYEYSMDY | 101 | 2 | 1 | IGKV14-111 | IGKJ1 | LQYDEFPRT | 104 | 4 | 3 | 4 | 1535 | 3562 | 4292 | 60.7 | 64.8 | ND | ND | ND |
| 195 | IGHV14-3 | IGHD2-3 | IGHJ4 | TRLDYDGYYEYALDY | 102 | 7 | 6 | IGKV14-111 | IGKJ1 | LQYDEFPRT | 104 | 4 | 1 | 4 | 584 | 1252 | 1640 | 56.9 | 80.1 | >10 | ND | >10 |
| 196 | IGHV14-3 | IGHD2-3 | IGHJ4 | ARLDYDAYYEYAMDY | 88 | 7 | 5 | IGKV14-111 | IGKJ1 | LQYDDFPRT | 105 | 2 | 0 | 4 | 4489 | 6378 | 11804 | 57.5 | 69.3 | 2.29 | 3.06 | >10 |
| 189-2 | IGHV14-3 | IGHD2-3 | IGHJ4 | ARLDYDGYYAMDY | 96 | 3 | 3 | IGKV14-111 | IGKJ1 | LQYDEFPRT | 104 | 6 | 4 | 4 | 5126 | 7614 | 9428 | 77.5 | 428 | 3.06 | >10 | ND |
| 184 | IGHV5-6-5 | IGHD2-4 | IGHJ3 | ARDGGDYDWFAY | 103 | 8 | 5 | IGKV12-44 | IGKJ1 | QHHYGSST | 106 | 6 | 5 | 3 | 456 | 69.4 | 964 | 560 | 63.3 | ND | ND | ND |
| Control | | | | | | | | | | | | | | | | | | | | | | |
| S309 (class 3) | NA | NA | NA | NA | | NA | NA | NA | NA | NA | | NA | NA | 3 | 10454 | 7315 | 8374 | 69.3 | 561 | 0.035 | 0.157 | 0.520 |
| R3022 (class | NA | NA | NA | NA | | NA | NA | NA | NA | NA | | NA | NA | 4 | 16476 | 22254 | 25842 | 12894 | 72 | 1.557 | 3.23 | >10 |

Fig. 13

| SARS-CoV-2.seq |
| Pangolin-GX-P2V.seq |
| SARS-CoV-1.seq |
| WIV1.seq |
| SHC014.seq |
| Anlong-103.seq |
| Rf1-2004.seq |
| Rs4081.seq |
| BM48-31.seq |

```
  1 NITNLCPFGEVFNATRFASVYAWNRKRISNCVADYSVLYNSA-SFSTFKCYGVSPTKLND      59
  1 ................SK...................T-....................      59
  1 ...............K.P.....E..K...........T-F.........A.....      59
  1 ...............T.P.....E.............T-.........A.....      59
  1 ...............T.P.....E.............T-.........A.....      59
  1 ....R...DSI...S..P.....E.TK..D.....T.....T-...........S..I.      59
  1 ........DK.......P.....E.TK..D....T.F...T-.....W......S..I.      59
  1 ....R...DK....S..PN....E.TK..D.....T.....T-............S..I.      59
  1 ...Q....N....I.S.P.....E.M..T...........SA.....Q...........      60
    ***..***...**.. *..****.*..* .*****.*.***. ****.*****...**.*
```

```
 60 LCFTNVYADSFVIRGDEVRQIAPGQTGKIADYNYKLPDDFTGCVIAWNSNNLDSKVGGNY     119
 60 .............VK...........V.................VKQ.ALT....     119
 60 ...S.........VK..D........V...........M...L...TR.I.ATST...     119
 60 ...S.........VK..D........V.............L...TR.I.ATQT...     119
 60 ...S.........VK..D........V............L...L...T.SK..STS...     119
 60 ....S....T.L..FS....V...E..V......R...........TA.Q.VGS----     115
 60 ....S....T.L..FS....V...V.............TAKQ.VGS----     115
 60 ....S....T.L..SS....V...E..V...........TAKQ.QGQ----     115
 61 ...SS....Y..VK..D......A...V.............T.S...SN----     116
    ***..****.*.......***.**..** ******.*****.***.***.  *  ...*
```

```
120 NYLYRLFRKSNLKPFERDISTEIYQAGSTPCNGVEGFNCYFPLQSYGFQPTNGVGYQPYR     179
120 G..........K...................QV.L...Y..ER...H..T..N...F.     179
120 ..K..YL.HGX.R.......NVPFSPDGK..TPP-AL...W..ND...YT.T.I.....     178
120 ..K..SL.HGX.R.......NVPFSPDGK..TPP-A....W..ND...YI...I.....     178
120 .....WV.R.K.N.Y...L.ND..SP.GQS.SA.-.P...N..RP...FT.A...H...     178
116 -.F..SH.STK.......L.S---------------DE.GVRT.ST.D.N.YVPLD..AT.     160
116 -.F..SH.S.K.......L.S---------------EE.GVRT.ST.D.MQNVPLE..AT.     160
116 -.Y..SS..TK.......LTS---------------DE.GVRT.ST.D.Y.NVPIE..AT.     160
117 EFF..R..HGKI..YG..L.NVLFNPSGGT.SAE-.L...K..A....TQSS.I.F....     175
      .**  *  ...*.**..        *..  *  *.*   . . .*..*
```

```
180 VVVLSFELLHAPATVCGPKK     199
180 .........NG........L     199
179 .........N.........L     198
179 .........N.........L     198
179 .........N.........L     198
161 .........N.........L     180
161 .........N.........L     180
161 .........N.........L     180
176 .........N.........Q     195
    *********..*********.
```

Fig. 14

*Sarbecovirus*

Fig. 15

MFVFLVLLPLVSSQCVNLTTRTQLPPAYTNSFTRGVYYPDKVFRSSVLHSTQDLFLPFFSN
VTWFHAIHVSGTNGTKRFDNPVLPFNDGVYFASTEKSNIIRGWIFGTTLDSKTQSLLIVNN
ATNVVIKVCEFQFCNDPFLGVYYHKNNKSWMESEFRVYSSANNCTFEYVSQPFLMDLE
GKQGNFKNLREFVFKNIDGYFKIYSKHTPINLVRDLPQGFSALEPLVDLPIGINITRFQTLLA
LHRSYLTPGDSSSGWTAGAAAYYVGYLQPRTFLLKYNENGTITDAVDCALDPLSETKCTLK
SFTVEKGIYQTSNFRVQPTESIVRFP**NITNLCPFGEVFNATRFASVYAWNRKRISNCVA**
**DYSVLYNSASFSTFKCYGVSPTKLNDLCFTNVYADSFVIRGDEVRQIAPGQTGKIAD**
**YNYKLPDDFTGCVIAWNSNNLDSKVGGNYNYLYRLFRKSNLKPFERDISTEIYQAG**
**STPCNGVEGFNCYFPLQSYGFQPTNGVGYQPYRVVVLSFELLHAPATVCGPKK**STN
LVKNKCVNFNFNGLTGTGVLTESNKKFLPFQQFGRDIADTTDAVRDPQTLEILDITPCSFG
GVSVITPGTNTSNQVAVLYQDVNCTEVPVAIHADQLTPTWRVYSTGSNVFQTRAGCLIG
AEHVNNSYECDIPIGAGICASYQTQTNSPRRARSVASQSIIAYTMSLGAENSVAYSNNSIA
IPTNFTISVTTEILPVSMTKTSVDCTMYICGDSTECSNLLLQYGSFCTQLNRALTGIAVEQD
KNTQEVFAQVKQIYKTPPIKDFGGFNFSQILPDPSKPSKRSFIEDLLFNKVTLADAGFIKQY
GDCLGDIAARDLICAQKFNGLTVLPPLLTDEMIAQYTSALLAGTITSGWTFGAGAALQIPF
AMQMAYRFNGIGVTQNVLYENQKLIANQFNSAIGKIQDSLSSTASALGKLQDVVNQNA
QALNTLVKQLSSNFGAISSVLNDILSRLDKVEAEVQIDRLITGRLQSLQTYVTQQLIRAAEI
RASANLAATKMSECVLGQSKRVDFCGKGYHLMSFPQSAPHGVVFLHVTYVPAQEKNFT
TAPAICHDGKAHFPREGVFVSNGTHWFVTQRNFYEPQIITTDNTFVSGNCDVVIGIVNN
TVYDPLQPELDSFKEELDKYFKNHTSPDVDLGDISGINASVVNIQKEIDRLNEVAKNLNES
LIDLQELGKYEQYIKWPWYIWLGFIAGLIAIVMVTIMLCCMTSCCSCLKGCCSCGSCCKFD
EDDSEPVLKGVKLHYT

## INTERNATIONAL SEARCH REPORT

International application No.

**PCT/JP2022/018507**

### A. CLASSIFICATION OF SUBJECT MATTER

*C07K 14/165*(2006.01)i; *A61K 39/215*(2006.01)i; *C12N 15/50*(2006.01)i
FI:   C07K14/165; A61K39/215; C12N15/50 ZNA

According to International Patent Classification (IPC) or to both national classification and IPC

### B. FIELDS SEARCHED

Minimum documentation searched (classification system followed by classification symbols)

C07K14/165; A61K39/215; C12N15/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/BIOSIS (STN); UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| P, X | SHINNAKASU, R. et al. Glycan engineering of the SARS-CoV-2 receptor-binding domain elicits cross-neutralizing antibodies for SARS-related viruses. Journal of Experimental Medicine. e20211003, vol. 218, no. 12, pp. 1-15 and S1-S4 [Online], 08 October 2021 [retrieved on 25 May 2022], Retrieved from the Internet:<URL:https://doi.org/10.1084/jem.20211003><DOI:10.1084/jem.20211003><br>whole document | 1-11 |
| A | QUINLAN, B. D., et al. An engineered receptor-binding domain improves the immunogenicity of multivalent SARS-CoV-2 vaccines. bioRxiv preprint. <DOI:https://doi.org/10.1101/2020.11.18.388934>, 18 November 2020, pp. 1-21 and supplementary materials [online], [retrieved on 25 May 2022], Retrieved from the Internet :<DOI:https://doi.org/10.1101/2020.11.18.388934><br>abstract, fig. 2, 3, S3, S4 | 1-11 |
| A | CASALINO, L. et al. Beyond shielding: the roles of glycans in the SARS-CoV-2 spike protein. ACS Central Science. 2020, vol. 6, pp.1722-1734 [online], [retrieved on 25 May 2022], [Retrieved from the Internet:<URL:http://dx.doi.org/10.1021/acscentsci.0c01056><br>abstract, fig. 1, 4 | 1-11 |

☑ Further documents are listed in the continuation of Box C.          ☐ See patent family annex.

| | | |
|---|---|---|
| \* | Special categories of cited documents: | "T" later document published after the international filing date or priority date and not in conflict with the application but cited to understand the principle or theory underlying the invention |
| "A" | document defining the general state of the art which is not considered to be of particular relevance | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **26 May 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/018507**

**C.    DOCUMENTS CONSIDERED TO BE RELEVANT**

| Category* | Citation of document, with indication, where appropriate, of the relevant passages | Relevant to claim No. |
|---|---|---|
| A | GURUPRASAD, L. Human SARS CoV-2 spike protein mutations. Proteins. vol. 89, pp. 569-576, 09 January 2021 [online], [retrieved on 25 May 2022], Retrieved from the Internet:<URL:https/onlinelibrary.wiley.com/doi/10.1002/prot.26042>, <DOI:10.1002/ prot.26042> <br>     abstract, table 3 | 1-11 |

Form PCT/ISA/210 (second sheet) (January 2015)

**INTERNATIONAL SEARCH REPORT**

International application No.

**PCT/JP2022/018507**

Box No. I    Nucleotide and/or amino acid sequence(s) (Continuation of item 1.c of the first sheet)

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **WACHARAPLUESADEE, S. ; TAN, C.W. ; MANEEOM, P. ; DUENGKAE, P. ; ZHU, F. ; JOYJINDA, Y. ; KAEWPOM, T. ; CHIA, W.N. ; AMPOOT, W. ; LIM, B.L. et al.** Evidence for SARS-CoV-2 related coronaviruses circulating in bats and pangolins in Southeast Asia. *Nat Commun,* 2021, vol. 12, 972
- **MENACHERY, V.D. ; B.L. YOUNT JR. ; K. DEBBINK ; S. AGNIHOTHRAM ; L.E. GRALINSKI ; J.A. PLANTE ; R.L. GRAHAM ; T. SCOBEY ; X.Y. GE ; E.F. DONALDSON et al.** A SARS-like cluster of circulating bat coronaviruses shows potential for human emergence. *Nat. Med.,* 2015, vol. 21, 1508-1513
- **GARCIA-BELTRAN, W.F. ; LAM, E.C. ; ASTUDILLO, M.G. ; YANG, D. ; MILLER, T.E. ; FELDMAN, J. ; HAUSER, B.M. ; CARADONNA, T.M. ; CLAYTON, K.L. ; NITIDO, A.D. et al.** COVID-19-neutralizing antibodies predict disease severity and survival. *Cell,* 2021, vol. 184, 476-488e411
- **SHANG et al.** Structural basis of receptor recognition by SARS-CoV-2. *Nature,* 2020, vol. 581, 221-224
- **JARDINE et al.** Rational HIV immunogen design to target specific germline B cell receptors. *Science,* 2013, vol. 340, 711-716
- **IMPAGLIAZZO et al.** A stable trimeric influenza hemagglutinin stem as a broadly protective immunogen. *Science,* 2015, vol. 349, 1301-1306
- **VALKENBURG et al.** Stalking influenza by vaccination with pre-fusion headless HA mini-stem. *Sci Rep,* 2016, vol. 6, 22666
- **DUAN et al.** Glycan Masking Focuses Immune Responses to the HIV-1 CD4-Binding Site and Enhances Elicitation of VRC01-Class Precursor Antibodies. *Immunity,* 2018, vol. 49, 301-311e305
- **EGGINK et al.** Guiding the immune response against influenza virus hemagglutinin toward the conserved stalk domain by hyperglycosylation of the globular head domain. *J Virol,* 2014, vol. 88, 699-704
- *Nucleic Acids Res,* 1994, vol. 22, 4673-4680
- Current Protocols in Molecular Biology. 2000
- Current Protocols in Molecular Biology. John Wiley and Sons, 1987
- **PINTO et al.** Cross-neutralization of SARS-CoV-2 by a human monoclonal SARS-CoV antibody. *Nature,* 2020, vol. 583, 290-295
- **YUAN et al.** Structural basis of a shared antibody response to SARS-CoV-2. *Science,* 2020, vol. 369, 1119-1123
- **ZHOU et al.** Structural basis for the neutralization of SARS-CoV-2 by an antibody from a convalescent patient. *Nat Struct Mol Biol,* 2020, vol. 27, 950-958
- **TAJIRI-TSUKADA et al.** Establishment of a highly precise multi-attribute method for the characterization and quality control of therapeutic monoclonal antibodies. *Bioengineered,* 2020, vol. 11, 984-1000
- **VON BOEHMER et al.** Sequencing and cloning of antigen-specific antibodies from mouse memory B cells. *Nat Protoc,* 2016, vol. 11, 1908-1923
- **INOUE et al.** Exit from germinal center to become quiescent memory B cells depends on metabolic reprograming and provision of a survival signal. *J Exp Med,* 2020, 218
- **WANG et al.** Anti-HA Glycoforms Drive B Cell Affinity Selection and Determine Influenza Vaccine Efficacy. *Cell,* 2015, vol. 162, 160-169
- **TANI et al.** Involvement of ceramide in the propagation of Japanese encephalitis virus. *J Virol,* 2010, vol. 84, 2798-2807
- **YOSHIDA et al.** SARS-CoV-2-induced humoral immunity through B cell epitope analysis in COVID-19 infected individuals. *Sci Rep,* 2021, vol. 11, 5934
- **NIE et al.** Quantification of SARS-CoV-2 neutralizing antibody by a pseudotyped virus-based assay. *Nat Protoc,* 2020, vol. 15, 3699-3715
- **MATSUDA et al.** High-throughput neutralization assay for multiple flaviviruses based on single-round infectious particles using dengue virus type 1 reporter replicon. *Sci Rep,* 2018, vol. 8, 16624
- **YAMANAKA et al.** A mouse monoclonal antibody against dengue virus type 1 Mochizuki strain targeting envelope protein domain II and displaying strongly neutralizing but not enhancing activity. *J Virol,* 2013, vol. 87, 12828-12837
- **GUPTA et al.** Change-O: a toolkit for analyzing large-scale B cell immunoglobulin repertoire sequencing data. *Bioinformatics,* 2015, vol. 31, 3356-3358
- **BARNES et al.** Structures of Human Antibodies Bound to SARS-CoV-2 Spike Reveal Common Epitopes and Recurrent Features of Antibodies. *Cell,* 2020, vol. 182, 828-842e816

- **STARR et al.** Deep Mutational Scanning of SARS-CoV-2 Receptor Binding Domain Reveals Constraints on Folding and ACE2 Binding. *Cell,* 2020, vol. 182, 1295-1310e1220
- **SHI et al.** A human neutralizing antibody targets the receptor-binding site of SARS-CoV-2. *Nature,* 2020, vol. 584, 120-124
- **BREITLING ; AEBI.** N-linked protein glycosylation in the endoplasmic reticulum. *Cold Spring Harb Perspect Biol,* 2013, vol. 5, a013359
- **ROBBIANI et al.** Convergent antibody responses to SARS-CoV-2 in convalescent individuals. *Nature,* 2020, vol. 584, 437-442
- **WALLS et al.** Elicitation of Potent Neutralizing Antibody Responses by Designed Protein Nanoparticle Vaccines for SARS-CoV-2. *Cell,* 2020, vol. 183, 1367-1382e1317
- **SATO et al.** Repurposing the psoriasis drug Oxarol to an ointment adjuvant for the influenza vaccine. *Int Immunol,* 2020, vol. 32, 499-507
- **CHEN et al.** Resistance of SARS-CoV-2 variants to neutralization by monoclonal and serum-derived polyclonal antibodies. *Nat. Med.,* 2021, vol. 27, 717-726
- **SMATTI et al.** Viral-Induced Enhanced Disease Illness. *Front Microbiol,* 2018, vol. 9, 2991
- **VERMA et al.** Analysis of the Fc gamma receptor-dependent component of neutralization measured by anthrax toxin neutralization assays. *Clin Vaccine Immunol,* 2009, vol. 16, 1405-1412
- **BAJIC et al.** Influenza Antigen Engineering Focuses Immune Responses to a Subdominant but Broadly Protective Viral Epitope. *Cell Host Microbe,* 2019, vol. 25, 827-835e826
- **LEACH et al.** Requirement for memory B-cell activation in protection from heterologous influenza virus reinfection. *Int Immunol,* 2019, vol. 31, 771-779
- **LAU et al.** Possible Bat Origin of Severe Acute Respiratory Syndrome Coronavirus 2. *Emerg Infect Dis,* 2020, vol. 26, 1542-1547
- **LETKO et al.** Functional assessment of cell entry and receptor usage for SARS-CoV-2 and other lineage B betacoronaviruses. *Nat Microbiol,* 2020, vol. 5, 562-569